(19) Europäisches Patentamt
European Patent Office
Office européen des brevets

(11) Veröffentlichungsnummer : **0 156 191 B1**

(12) **EUROPÄISCHE PATENTSCHRIFT**

(45) Veröffentlichungstag der Patentschrift : **19.07.89**

(21) Anmeldenummer : **85102387.9**

(22) Anmeldetag : **04.03.85**

(51) Int. Cl.$^4$ : **C 07 D487/04**, C 07 D471/04, C 07 D243/38, C 07 D495/04, A 61 K 31/55// C07D207/16, C07D207/40, C07D211/26, C07D223/08, C07D223/04 ,(C07D487/04, 243:00, 209:00),(C07D495/04, 333:00, 243:00),(C07D471/04, 243:00, 221:00)

# (54) Kondensierte Diazepinone, Verfahren zu ihrer Herstellung und diese Verbindungen enthaltende Arzneimittel.

(30) Priorität : **14.03.84 DE 3409237**

(43) Veröffentlichungstag der Anmeldung : **02.10.85 Patentblatt 85/40**

(45) Bekanntmachung des Hinweises auf die Patenterteilung : **19.07.89 Patentblatt 89/29**

(84) Benannte Vertragsstaaten : **AT BE CH DE FR GB IT LI LU NL SE**

(56) Entgegenhaltungen :
EP-A- 0 039 519
EP-A- 0 044 989
EP-A- 0 057 428
FR-A- 2 016 009

(73) Patentinhaber : **Dr. Karl Thomae GmbH**
**Postfach 1755**
**D-7950 Biberach (Riss) (DE)**

(72) Erfinder : **Engel, Wolfhard, Dr. Dipl.-Chem.**
**Mozartstrasse 13**
**D-7950 Biberach 1 (DE)**
Erfinder : **Trummlitz, Günter, Dr. Dipl.-Chem.**
**Buchenweg 27**
**D-7951 Warthausen (DE)**
Erfinder : **Eberlein, Wolfgang, Dr. Dipl.-Chem.**
**Obere Au 6**
**D-7950 Biberach 1 (DE)**
Erfinder : **Mihm, Gerhard, Dr. Dipl.-Chem.**
**Nickeleshalde 5/1**
**D-7950 Biberach 1 (DE)**
Erfinder : **Schmidt, Günther, Dr. Dipl.-Chem.**
**Johann-Seb.-Bach-Strasse 27**
**D-7950 Biberach 1 (DE)**
Erfinder : **Hammer, Rudolf, Dr.**
**Grundstrasse 64**
**D-6507 Ingelheim/Rhein (DE)**
Erfinder : **Giachetti, Antonio, Prof. Dr.**
**Guerrini 3**
**Milano (IT)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 156 191 B1

Jouve, 18, rue St-Denis, 75001 Paris, France

**Beschreibung**

Die Erfindung betrifft neue kondensierte Diazepinone, Verfahren zu ihrer Herstellung und diese Verbindungen enthaltende Arzneimittel.

Aus EP-A-0 039 519 und 0 057 428 sowie aus US-A 3 660 380 ; 3 691 159 ; 4 213 984 ; 4 213 985 ; 4 210 648, 4 410 527 ; 4 424 225 ; 4 424 222 und 4 424 226 sind bereits kondensierte Diazepinone mit ulcushemmenden und magensaftsekretionshemmenden Eigenschaften bekannt.

Es wurde nun gefunden, daß die erfindungsgemäßen Diazepinone mit neuartigen Aminoacylresten gegenüber den Verbindungen der obengenannten Publikationen überraschenderweise völlig andersartige, wertvolle pharmakologische Eigenschaften aufweisen.

Die neuen kondensierten Diazepinone besitzen die allgemeine Formel I

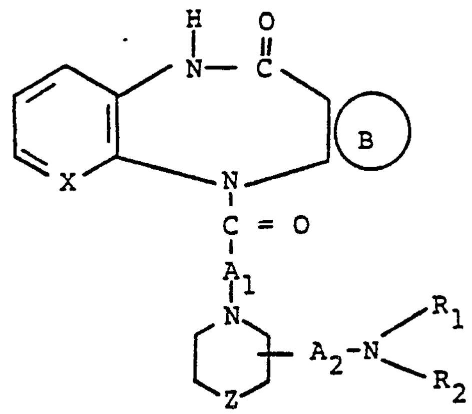

(I)

in der

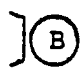

einen der zweiwertigen Reste

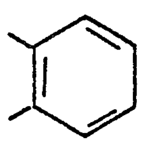 , 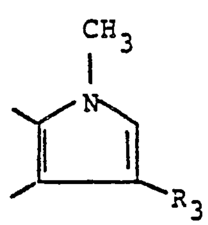 oder 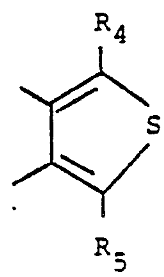

bedeutet und

X, $A_1$, $A_2$, $R_1$ bis $R_5$ und Z die folgenden Bedeutungen besitzen :

X ist die =CH-Gruppe oder, sofern

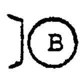

den ortho-Phenylenrest darstellt, auch ein Stickstoffatom ;

$A_1$ ist ein Alkylenrest mit 1 bis 2 Kohlenstoffatomen,

$A_2$ entweder ein Alkylenrest mit 1 bis 2 Kohlenstoffatomen, sofern er sich in der 2-Stellung zum Stickstoff des gesättigten heterocyclischen Ringes befindet oder, wenn er sich in der 3- oder 4-Stellung befindet, eine Einfachbindung oder die Methylengruppe,

$R_1$ ist ein verzweigter oder unverzweigter Alkylrest mit 1 bis 3 Kohlenstoffatomen,

$R_2$ ein verzweigter oder unverzweigter Alkylrest mit 1 bis 7 Kohlenstoffatomen, der gegebenenfalls noch an seinem 2. bis 7. Kohlenstoffatom durch eine Hydroxygruppe substituiert sein kann, ein

Cycloalkyl- oder ein Cycloalkylmethylrest mit 3 bis 7 Kohlenstoffatomen im Ring, wobei der Cycloalkylring gegebenenfalls noch durch eine Hydroxygruppe substituiert sein kann,

$R_1$ und $R_2$ können aber auch zusammen mit dem dazwischenliegenden Stickstoffatom einen 4- bis 7-gliedrigen gesättigten, monocyclischen, heterocyclischen Ring bilden, der gegebenenfalls durch ein Sauerstoffatom oder durch die N—$CH_3$-Gruppe unterbrochen sein kann,

$R_3$ ist ein Wasserstoff- oder Chloratom oder die Methylgruppe,

$R_4$ ist ein Wasserstoffatom oder ein Alkylrest mit 1 bis 4 Kohlenstoffatomen,

$R_5$ ist ein Wasserstoff- oder Chloratom oder ein Alkylrest mit 1 bis 4 Kohlenstoffatomen und

Z entweder eine Einfachbindung oder ein Sauerstoffatom oder die Methylen- oder 1,2-Ethylengruppe.

Bevorzugte Verbindungen der obigen allgemeinen Formel I sind diejenigen, in denen

$R_1$ bis $R_3$ und X wie eingangs definiert sind,

Z eine Einfachbindung oder eine Methylengruppe,

$A_1$ und $A_2$ jeweils eine Methylengruppe,

$R_4$ ein Wasserstoffatom oder eine Methylgruppe und

$R_5$ ein Wasserstoff- oder Chloratom oder eine Methylgruppe bedeuten, und

besonders bevorzugte Verbindungen der obigen allgemeinen Formel I, in denen

X wie eingangs definiert ist,

Z eine Einfachbindung oder eine Methylengruppe,

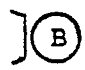

eine ortho-Phenylengruppe oder eine 3,4-verknüpfte Thieno- gruppe,

$A_1$ eine Methylengruppe,

$A_2$ eine Methylengruppe in 2-Stellung zum N-Atom des gesättigten heterocyclischen Rings,

$R_1$ eine Methyl- oder Ethylgruppe und

$R_2$ eine Methyl-, Ethyl- oder 4-Hydroxycyclohexylgruppe bedeuten.

Gewünschtenfalls können die Verbindungen der allgemeinen Formel I auch nachträglich in ihre $NR_1R_2$—N-Oxide überführt werden.

Die Verbindungen der allgemeinen Formel I können nach Umsetzung mit anorganischen oder organischen Säuren auch in Form ihrer physiologisch verträglichen Salze vorliegen. Als Säuren haben sich beispielsweise Salzsäure, Bromwasserstoffsäure, Schwefelsäure, Methylschwefelsäure, Phosphorsäure, Weinsäure, Fumarsäure, Zitronensäure, Maleinsäure, Bernsteinsäure, Gluconsäure, Äpfelsäure, p-Toluolsulfonsäure, Methansulfonsäure oder Amidosulfonsäure als geeignet erwiesen.

Zur Erläuterung des Erfindungsgegenstandes seien als Beispiele folgende Verbindungen genannt :

11-[[2-[(Diethylamino)methyl]-1-piperidinyl]acetyl]-5,11-dihydro-6H-pyrido[2,3-b] [1,4] benzodiazepin-6-on

5,11-Dihydro-11-[[2-(dimethylamino)methyl]-1-piperidinyl]acetyl]-6H-pyrido[2,3-b] [1,4] benzodiazepin-6-on

5,11-dihydro-11[[2-[2-(dimethylamino)ethyl]-1-piperidinyl]acetyl]-6H-pyrido[2,3-b] [1,4] benzodiazepin-6-on

5-[[2-[(Diethylamino)methyl]-1-piperidinyl]acetyl]-5,10-dihydro-11H-dibenzo[b,e] [1,4] diazepin-11-on

4-[[2-[(Diethylamino)methyl]-1-piperidinyl]acetyl]4,9-dihydro-10H-thieno[3,4-b] [1,5] benzodiazepin-10-on

(S)-11-[[2-[[Diethylamino)methyl]-1-pyrrolidinyl]acetyl]-5,11-dihydro-6H-pyrido[2,3-b] [1,4] benzodiazepin-6-on

(S)-5-[[2-[(Diethylamino)methyl]-1-pyrrolidinyl]acetyl]-5,10-dihydro-11H-dibenzo[b,e] [1,4] diazepin-11-on

trans-5,11-Dihydro-11-[[2-[[(4-hydroxycyclohexyl)-(methyl)amino]methyl]-1-piperidinyl]acetyl]-6H-pyrido[2,3-b] [1,4] benzodiazepin-6-on

11-[[3-[[(Cyclohexyl) (methyl)amino]methyl]-1-piperidinyl]acetyl]-5,11-dihydro-6H-pyrido[2,3-b] [1,4] benzodiazepin-6-on

5-[[3-[(Diethylamino)methyl]-1-piperidinyl]acetyl]-5,10-dihydro-11H-dibenzo[b,e] [1,4] diazepin-11-on

5-[[2-[[(Cyclohexyl)methyl)amino]methyl]-1-piperidinyl]acetyl]-5,10-dihydro-11H-dibenzo[b,e] [1,4]diazepin-11-on

5-[[3-[[(Cyclohexyl) (methyl)amino]methyl]-1-piperidinyl]-acetyl]-5,10-dihydro-11H-dibenzo[b,e] [1,4]diazepin-11-on

5,10-Dihydro-5-[[3-(1-methyl-2-pyrrolidinyl)-1-piperidinyl]acetyl]-11H-dibenzo[b,e] [1,4] diazepin-11-on

11-[[2-[(Butylmethylamino)methyl]-1-piperidinyl]acetyl]-5,10-dihydro-11H-dibenzo[b,e] [1,4]) diazepin-11-on

Gegenstand der Erfindung sind darüber hinaus die als Zwischenprodukte verwendeten neuen Pyrrolo-benzodiazepinone der allgemeinen Formel Ia

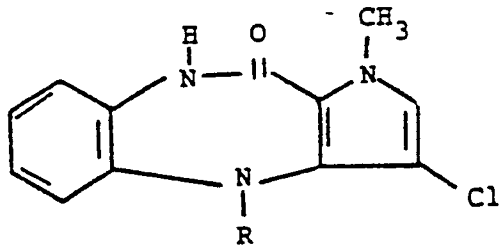

(Ia)

in der R das Wasserstoffatom oder einen Halogenacylrest, vorzugsweise einen Chloracylrest, mit insgesamt 2 oder 3 Kohlenstoffatomen bedeutet.

Erfindungsgemäß erhält man die neuen basisch substituierten kondensierten Diazepinone der allgemeinen Formel I nach folgenden Verfahren :

a) Basisch substituierte kondensierte Diazepinone der allgemeinen Formel Ib,

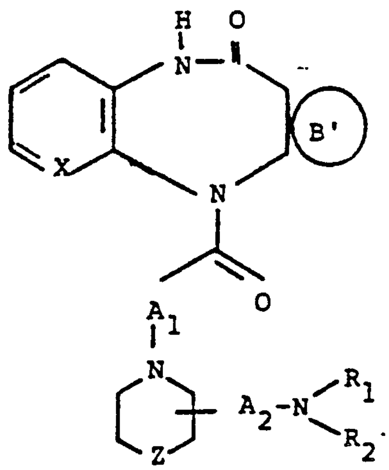

(Ib)

in der $R_1$, $R_2$, X, Z, $A_1$ und $A_2$ die oben angegebene Bedeutung haben und

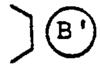

einen der zweiwertigen Reste

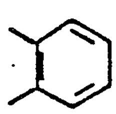 , 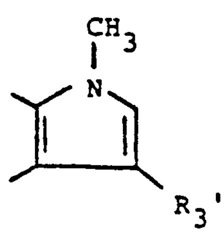

oder

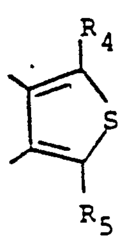

darstellt, wobei $R_4$ und $R_5$ wiederum die eingangs gegebenen Bedeutungen annehmen können und $R_3'$ die Methylgruppe oder das Chloratom ist, erhält man durch Umsetzung von Halogenacylverbindungen der allgemeinen Formel II

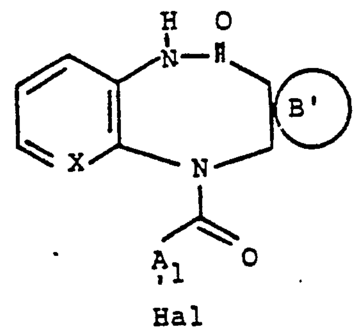

(II)

in der X,

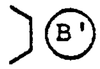

und $A_1$ die angegebenen Bedeutungen haben und Hal ein Chlor-, Brom- oder Jodatom ist, mit sekundären Aminen der allgemeinen Formel III,

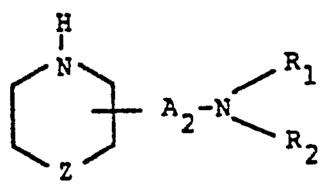

(III)

in der $R_1$, $R_2$, $A_2$ und Z die eingangs definierten Bedeutungen besitzen.

Die Aminierung erfolgt in einem inerten Lösungsmittel bei Temperaturen zwischen — 10 °C und der Siedetemperatur des Lösungsmittels, vorzugsweise entweder mit wenigstens 2 Molen sekundärem Amin der allgemeinen Formel III oder mit 1 bis 2 Molen eines sekundären Amins der allgemeinen Formel III und einer Hilfsbase. Als Lösungsmittel kommen beispielsweise chlorierte Kohlenwasserstoffe, wie Methylenchlorid, Chloroform oder Dichlorethan ; offenkettige oder cyclische Ether, wie Diethylether, Tetrahydrofuran oder Dioxan ; aromatische Kohlenwasserstoffe, wie Benzol, Toluol, Xylol, Chlorbenzol oder Pyridin ; Alkohole, wie Ethanol oder Isopropanol ; Ketone, wie Aceton ; Acetonitril oder Dimethylformamid in Frage. Als Hilfsbasen seien beispielsweise tertiäre organische Basen, wie Triethylamin, N-Methylpiperidin, Diethylanilin, Pyridin und 4-(Dimethylamino)pyridin oder anorganische Basen, wie Alkalimetall- oder Erdalkalimetallcarbonate oder -hydrogencarbonate, -hydroxide oder -oxide genannt. Gegebenenfalls kann die Reaktion durch Zusatz von Alkalimetalliodiden beschleunigt werden. Die Reaktionszeiten betragen je nach Menge und Art des eingesetzten Amins der allgemeinen Formel III zwischen 15 Minuten und 80 Stunden.

Bei Umsetzung von Ausgangsverbindungen der allgemeinen Formel II, in denen $A_1$ eine Ethylengruppe darstellt, kann die Reaktion auch unter Abspaltung von H-Hal verlaufen ; die intermediär gebildete, gegebenenfalls isolierbare Acryloylverbindung reagiert mit dem sekundären Amin der allgemeinen Formel III zum gleichen Endprodukt der allgemeinen Formel Ib.

b) Verbindungen der allgemeinen Formel Ib können auch dadurch erhalten werden, daß man Tricyclen der allgemeinen Formel IV,

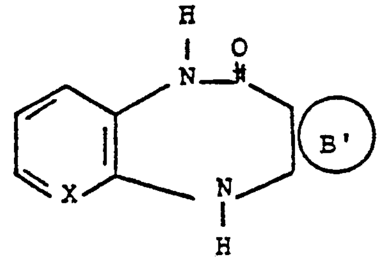

(IV)

worin

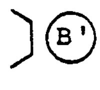

und X die oben angegebenen Bedeutungen haben, mit Carbonsäurederivaten der allgemeinen Formel V,

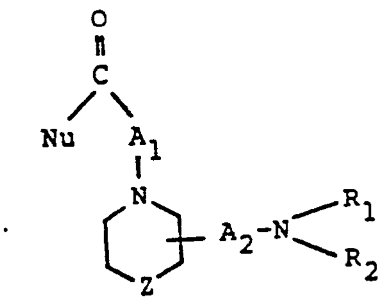

(V)

worin $R_1$, $R_2$, $A_1$, $A_2$ und Z die oben angegebene Bedeutung haben und Nu eine nucleofuge Gruppe bzw. Abgangsgruppe darstellt, acyliert.

Die Umsetzung der Verbindungen der allgemeinen Formel IV mit den Säurederivaten der allgemeinen Formel V erfolgt in an sich bekannter Weise. Die Abgangsgruppe Nu ist eine Gruppe, die gemeinsam mit der Carbonylgruppe, an die sie gebunden ist, ein reaktives Carbonsäurederivat bildet. Als reaktive Carbonsäurederivate seien beispielsweise Säurehalogenide, -ester, -anhydride oder gemischte -anhydride, wie sie aus Salzen der entsprechenden Säuren (Nu=OH) und Säurechloriden, wie Phosphoroxidchlorid, Diphosphorsäuretetrachlorid oder Chlorameisensäureestern gebildet werden oder die bei der Umsetzung von Verbindung der allgemeinen Formel V (Nu=OH) mit N-Alkyl-2-halogenpyridiniumsalzen entstehenden N-Alkyl-2-acyloxypyridiniumsalze genannt.

Bevorzugt wird die Reaktion mit den gemischten Anhydriden starker Mineralsäuren, insbesondere der Dichlorphosphorsäure, durchgeführt. Die Reaktion wird gegebenenfalls in Gegenwart eines säurebindenden Mittels (Protonenakzeptors) durchgeführt. Als geeignete Protonenakzeptoren seien beispielsweise Alkalimetallcarbonate oder -hydrogencarbonate, wie Natriumcarbonat oder Kaliumhydrogencarbonat; tertiäre organische Amine, wie Pyridin, Triethylamin, Ethyldiisopropylamin, 4-(Dimethylamino)pyridin, oder Natriumhydrid genannt. Die Reaktion wird bei Temperaturen zwischen — 25 °C und 130 °C in einem inerten Lösungsmittel durchgeführt. Als inerte Lösungsmittel kommen beispielsweise chlorierte aliphatische Kohlenwasserstoffe, wie Methylenchlorid, 1,2-Dichlorethan; offenkettige oder cyclische Ether, wie Diethylether, Tetrahydrofuran oder 1,4-Dioxan; aromatische Kohlenwasserstoffe, wie Benzol, Toluol, Xylol, o-Dichlorbenzol; polare aprotische Lösungsmittel wie Acetonitril, Dimethylformamid oder Hexamethylphosphorsäuretriamid; oder Gemische davon in Frage. Die Reaktionszeiten betragen je nach Menge und Art des eingesetzten Acylierungsmittels der allgemeinen Formel V zwischen 15 Minuten und 80 Stunden. Es ist nicht nötig, die Verbindungen der allgemeinen Formel V in reiner Form herzustellen, sie können vielmehr im Reaktionsansatz in bekannter Weise in situ erzeugt werden.

c) Die unter die allgemeine Formel I fallenden neuen Pyrrolobenzodiazepinone der allgemeinen Formel Ic,

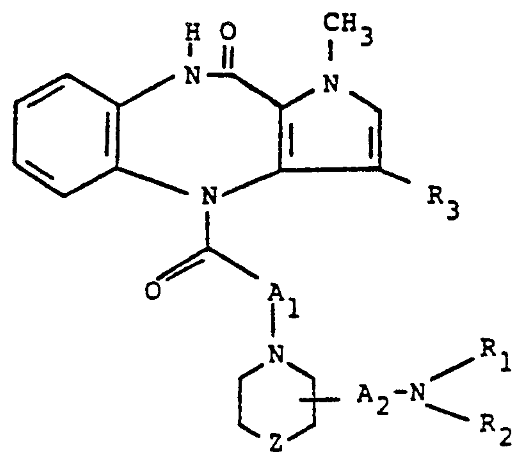

(Ic)

worin $R_1$, $R_2$, $A_1$, $A_2$ und Z die eingangs erwähnten Bedeutungen haben und $R_3$ ein Wasserstoffatom ist, werden durch Hydrogenolyse aus entsprechenden Verbindungen der allgemeinen Formel Ic, worin $R_3$ ein Chloratom bedeutet, hergestellt. Die Hydrogenolyse wird in Gegenwart von Katalysatoren von Metallen aus der VIII. Nebengruppe des Periodensystems der Elemente, beispielsweise von Palladium auf Tierkohle, Palladium auf Bariumsulfat, Raney-Nickel oder Raney-Cobalt, und bei Wasserstoffdrucken von 1 bis 300 bar und Temperaturen von 0 °C bis 130 °C in Gegenwart von Lösungsmitteln, beispielsweise Alkoholen, wie Methanol, Ethanol; Ethern wie Dioxan, Tetrahydrofuran, Carbonsäuren, beispielsweise Essigsäure oder tertiären Aminen, beispielsweise Triethylamin, durchgeführt. Arbeitet man dabei in

Abwesenheit zusätzlicher Chlorwasserstoff-Akzeptoren, beispielsweise von Natriumcarbonat, Kaliumhydrogencarbonat, Triethylamin oder Natriumacetat, so entstehen direkt die Hydrochloride der gesuchten Verbindungen, die nach Entfernung des Katalysators durch Eindampfen der Reaktionslösung isoliert werden können. Ersetzt man in der vorstehenden Hydrogenolyse-Reaktion den Wasserstoff durch Ameisensäure, so gelingt die Umsetzung prinzipiell schon bei drucklosem Arbeiten. Bei dieser Variante haben sich besonders die Umsetzung mit Ameisensäure in Gegenwart von Dimethylformamid als Lösungsmittel und von Palladium auf Kohle als Katalysator bei Temperaturen zwischen 70 und 110 °C, sowie die Reduktion mit Triethylammoniumformiat in Gegenwart überschüssigen Triethylamins und von Palladium auf Tierkohle oder von Palladiumacetat und Triarylphosphinen, wie Triphenylphosphin, Tris-(o-tolyl)phosphin, Tris-(2,5-diisopropylphenyl)phosphin, bei Temperaturen zwischen 40 und 110 °C, bewährt.

Die Verbindungen der allgemeinen Formel I können gewünschtenfalls nachträglich durch Oxidation mit Wasserstoffperoxid oder mit Persäuren, z. B. mit 3-Chlor-perbenzoesäure, in ihre $NR_1R_2$—N-Oxide überführt werden. Durch diese Oxidation wird nur das Stickstoffatom der Seitenkette des Ringes

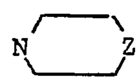

in das N-Oxid überführt.

So erhaltene Basen der allgemeinen Formel I können anschließend in ihre Säureadditionssalze oder erhaltene Säureadditionssalze in die freien Basen oder andere pharmakologisch verträgliche Säureadditionssalze übergeführt werden.

Die erfindungsgemäßen basisch substituierten kondensierten Diazepinone der allgemeinen Formel I enthalten bis zu zwei unabhängige chirale Elemente, davon ein asymmetrisches Kohlenstoffatom in der Seitenkette. Als zweites chirales Element ist der acylierte Tricyclus selbst anzusehen, der in zwei spiegelbildlichen Formen vorliegen kann. Von der Natur des Tricyclus hängt es ab, ob die Energiebarriere für eine Inversion an diesem Zentrum so hoch ist, daß die einzelnen Isomeren bei Zimmertemperatur stabil sind und isolierbar werden. Es hat sich gezeigt, daß solche Verbindungen der allgemeinen Formel I, in der X die CH-Gruppe ist und

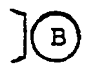

den o-Phenylenrest bedeutet, stets in 2 diastereomeren Formen vorliegen, die bei Zimmertemperatur in die Komponenten getrennt werden können. Die einzelnen Diastereomeren sind in kristallinem Zustand völlig stabil, gehen in Lösung und bei Zimmertemperatur aber mit einer Halbwertzeit von einigen Tagen wieder ins ursprüngliche Gemisch über. Bei Verbindungen der allgemeinen Formel I, in der X das N-Atom und

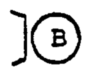

die o-Phenylengruppe bedeutet, ist die erforderliche Aktivierungsenergie so stark vermindert, daß Diastereomere bei Zimmertemperatur — außer durch die komplexen $^1H$-NMR-Spektren — nicht mehr nachgewiesen geschweige denn präparativ getrennt werden können.

Die erfindungsgemäßen aminoacylierten kondensierten Diazepinone der allgemeinen Formel I enthalten also in der Regel 2 chirale Zentren, von denen eines bei Zimmertemperatur unter Umständen nicht konfigurationsstabil ist. Diese Verbindungen können deshalb in zwei diastereomeren Formen oder jeweils als enantiomer (+)- und (—)-Formen auftreten. Die Erfindung umfaßt die einzelnen Isomeren ebenso wie ihre Gemische. Die Trennung der jeweiligen Diastereomeren gelingt auf Grund der unterschiedlichen physiko-chemischen Eigenschaften, z. B. durch fraktioniertes Umkristallisieren aus geeigneten Lösungsmitteln, durch Hochdruckflüssigkeitschromatographie, Säulenchromatographie oder gaschromatographische Verfahren.

Die Spaltung evtl. Razemate der Verbindungen der allgemeinen Formel I kann nach bekannten Verfahren durchgeführt werden, beispielsweise unter Verwendung einer optisch aktiven Säure, wie (+)- oder (—)-Weinsäure, oder eines Derivats davon, wie (+)- oder (—)-Diacetylweinsäure, (+)- oder (—)-Monomethyltartrat oder (+)-Camphersulfonsäure.

Nach einem üblichen Verfahren zur Isomerentrennung wird das Razemat einer Verbindung der allgemeinen Formel I mit einer der vorstehend angegebenen optisch aktiven Säuren in äquimolarer Menge in einem Lösungsmittel umgesetzt und die erhaltenen kristallinen optisch aktiven Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50 : 50, verwendet. Sodann wird jedes der optisch aktiven Salze in Wasser gelöst, mit

einer Base, wie Natriumcarbonat oder Kaliumcarbonat, neutralisiert und dadurch die entsprechende freie Verbindung in der (+)- oder (—)-Form erhalten.

Jeweils nur ein Enantiomeres bzw. ein Gemisch zweier optisch aktiver unter die allgemeine Formel I fallender diastereomerer Verbindungen wird auch dadurch erhalten, daß man die oben beschriebenen Synthesen mit nur einem Enantiomeren der allgemeinen Formel III bzw. V durchführt.

Die erfindungsgemäße und als Zwischenprodukt benötigte neue Verbindung der Formel Ia, in der R das Wasserstoffatom bedeutet, erhält man dadurch, daß man Verbindungen der allgemeinen Formel VI,

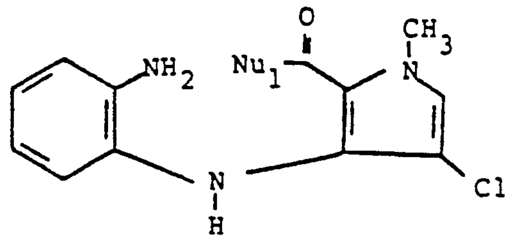

(VI)

in der $Nu_1$ eine geeignete nucleofuge Gruppe (=Abgangsgruppe) darstellt, cyclisiert und gegebenenfalls anschließend den erhaltenen Tricyclus der allgemeinen Formel Ia, in der R das Wasserstoffatom bedeutet, nach den nachstehend für die Synthese von Verbindungen der allgemeinen Formel II angebenen Verfahren acyliert, wodurch man solche Verbindungen der allgemeinen Formel Ia erhält, worin R einen Halogenacetyl- oder 3-Halogen-1-oxopropyl-Rest darstellt.

Die Cyclisierung von Verbindungen der allgemeinen Formel VI wird — je nach Art der nucleofugen Gruppe $Nu_1$ in an sich bekannter Weise durchgeführt. Eine geeignete nucleofuge Gruppe $Nu_1$ ist eine Gruppe, die zusammen mit der Carbonylgruppe, an die sie gebunden ist, ein reaktives Carbonsäurederivat bildet. Eine geeignete nucleofuge Gruppe $Nu_1$ ist beispielsweise eine Alkoxygruppe, eine Aminogruppe oder eine Hydroxygruppe.

Ist $Nu_1$ z. B eine Aminogruppe, so wird die Cyclisierung ohne Lösungsmittel oder in inerten, bevorzugt polaren organischen Lösungsmitteln, wie niederen Alkoholen, z. B. Ethanol, gegebenenfalls in Gegenwart einer Säure, wie Halogenwasserstoffsäure, oder in Gegenwart einer Base, z. B. eines Alkalimetallalkanolats, bei Temperaturen zwischen 0 °C und 200 °C, bevorzugt bei der Siedetemperatur des verwendeten Lösungsmittels durchgeführt. Ist $Nu_1$ eine Alkoxygruppe, so wird die Cyclisierung der Verbindungen VI bei Temperaturen zwischen 0 °C und 200 °C, vorzugsweise 20 und 120 °C, ohne Lösungsmittel oder in Gegenwart eines inerten Lösungsmittels, gegebenenfalls in Gegenwart eines basischen oder vorzugsweise sauren Katalysators durchgeführt. Die Reaktionszeiten betragen 15 Minuten bis 6 Stunden. Als Lösungsmittel kommen beispielsweise Alkohole, wie Ethanol, Isopropanol oder Glykol ; Ether, wie Dioxan oder Diphenylether ; aromatische Kohlenwasserstoffe, wie Toluol, Xylol oder o-Dichlorbenzol ; oder Dimethylsulfoxid in Frage. Man kann die Reaktion jedoch auch in Abwesenheit von zusätzlichen Lösungsmitteln durchführen.

Als Katalysatoren seien basische Katalysatoren, wie Alkalimetallalkanolate, z. B. Natriummethylat oder Kalium-tert.-butanolat, n-Butyllithium oder Natriumhydrid ; oder saure Katalysatoren, wie organische oder anorganische Säuren, z. B. Essigsäure, Chloressigsäure, p-Toluolsulfonsäure, o-Chlorbenzoesäure, p-Tolylsäure, Nikotinsäure, Trifluoressigsäure, Fumarsäure, Chlorwasserstoffsäure, Benzoesäure, Kaliumhydrogensulfat oder vorzugsweise Phosphorsäure genannt, wobei gegebenenfalls mehrere Mol saurer Katalysator je Mol Ausgangsverbindung eingesetzt werden.

Ist $Nu_1$ eine Hydroxygruppe, so erfolgt die Cyclisierung der Verbindung VI beispielsweise in polaren Lösungsmitteln, bevorzugt unter saurer Katalyse, und vorteilhafterweise in Gegenwart eines Kondensationsmittels, wie N,N'-Dicyclohexylcarbodiimid, oder unter kontinuierlicher Abscheidung des bei der Reaktion entstehenden Wassers, z. B. durch azeotropes Ausschleppen des Wassers am Wasserabscheider. Die Reaktion erfolgt bevorzugt bei Temperaturen zwischen 50 °C und 200 °C, insbesondere bei Temperaturen zwischen 50 °C und 160 °C.

Zu Verbindungen der Formel VI gelangt man durch Umsetzung von o-Halogennitrobenzolen der allgemeinen Formel VII, bevorzugt 2-Fluor-nitrobenzol, mit 3-Aminopyrrolen der allgemeinen Formel VIII

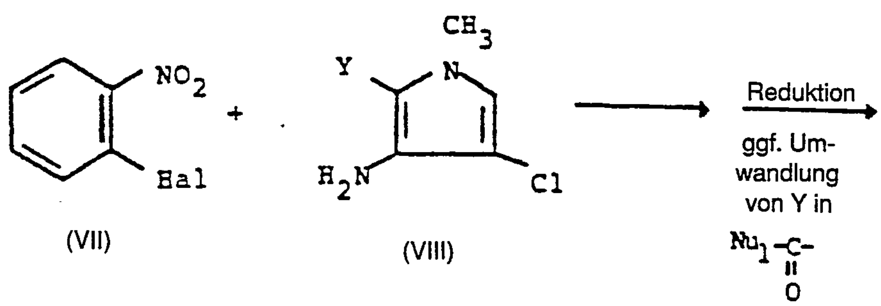


(VII) (VIII)

(wobei in der Formel VII Hal ein Halogenatom — insbesondere ein Fluor- oder Chloratom — und Y eine $Nu_1-C{\leqq}^{O}$-Gruppe oder den Vorläufer einer $Nu_1-C{\leqq}^{O}$-Gruppe darstellt), anschließende Reduktion der Nitrogruppe zur Aminogruppe und gegebenenfalls anschließende Umwandlung des Restes Y in einen geeigneten Rest $Nu_1-C{\leqq}^{O}$. Unter dem Vorläufer einer $Nu_1-C{\leqq}^{O}$-Gruppe (=Y) versteht man einen Substituenten, der durch einen geeigneten, dem Fachmann geläufigen Verfahrensschritt (z. B. Hydrolyse, Alkoholyse, Ansäuern, Verseifung etc.) in die $Nu_1-C{\leqq}^{O}$-Gruppe umgewandelt werden kann. Bevorzugte Vorläufer sind die Nitril-Gruppe (—CN) und die Carboxylat-Gruppe ($—COO^{\ominus}$), die durch Hydrolyse, Alkoholyse bzw. Ansäuern in die Amid-Gruppe, Ester-Gruppe bzw. in den Carbonsäurerest übergeführt werden.

Die Umsetzung der o-Halogennitrobenzole der allgemeinen Formel VIII mit den 3-Aminopyrrolen der allgemeinen Formel VIII erfolgt in an sich bekannter Weise, bevorzugt unter Zusatz eines Deprotonierungsmittels, z. B. Kalium-tert.-butanolat, Natriumhydrid, Kaliumcarbonat oder eines tertiären Amins, in Lösungsmitteln wie Dimethylformamid, Dioxan, Tetrahydrofuran oder N-Methylpyrrolidon, je nach Art des Restes Hal in Verbindungen der allgemeinen Formel VII und je nach Art des verwendeten Deprotonierungsmittels bei Temperaturen zwischen 0 °C und 150 °C.

Die Reduktion der Nitrogruppe zur Aminogruppe gelingt nach den bei Nitroaromaten bzw. Nitroheterocyclen üblichen Methoden.

Die Herstellung der Aminopyrrole der allgemeinen Formel VIII erfolgt durch Ringschluß von N-(2-Chlor-3-cyan-ethenyl)-methylamino-essigsäurederivaten bzw. acetonitrilen in Gegenwart starker Basen, z. B. von Kalium-tert.-butanolat, Natriumethanolat oder Natriumhydrid, und in geeigneten inerten Lösungsmitteln, beispielsweise Toluol, Dimethylformamid, N-Methylpyrrolidon. Die Aminopyrrole der allgemeinen Formel VIII brauchen nicht in reiner Form isoliert zu werden, sondern können — ohne Aufarbeitung der Reaktionslösungen — noch im gleichen Lösungsmittel mit den Halogennitrobenzolen der allgemeinen Formel VII weiter umgesetzt werden. Die N-(2-Chlor-2-cyan-ethenyl)-methylamino-essigsäurederivate, z. B. die entsprechenden Methyl- oder Ethylester, bzw. Acetonitrile können in einfacher Weise durch Addition von Sarcosinestern bzw. (Methylamino)acetonitril an 2,3-Dichloracrylnitril erhalten werden. 2,3-Dichloracrynitril braucht nicht als solches eingesetzt zu werden sondern kann in situ aus 2,2,3-Trichlorpropionitril in Gegenwart von Basen, z. B. von Triethylamin, erzeugt werden. 2,2,3-Trichlorpropionitril wiederum entsteht — entgegen anders lautenden Angaben in einem Patent (American Cyanamid Co., US-A 3 161 577 ; C.A. 62, 6399d[1965]) — als einziges Produkt bei der Photochlorierung von 3-Chlorpropionitril.

Zur Herstellung der Halogenacylverbindungen der allgemeinen Formel II werden die Ausgangsverbindungen der allgemeinen Formel IV mit Verbindungen der allgemeinen Formel IX Hal-$A_1$-CO-Hal' (IX) oder [Hal-$A_1$-CO]$_2$O (X), worin Hal' eine der Bedeutungen von Hal hat und $A_1$ und Hal die oben angegebenen Bedeutungen haben, umgesetzt. Diese Acylierung wird ohne oder vorzugsweise in einem inerten Lösungsmittel bei Raumtemperatur oder erhöhter Temperatur, maximal bei der Siedetemperatur des Lösungsmittels, gegebenenfalls in Gegenwart einer Hilfsbase und/oder eines Acylierungskatalysators, vorgenommen. Die Säurehalogenide der allgemeinen Formel IX sind gegenüber den Säureanhydriden der allgemeinen Formel X bevorzugt. Als Säurehalogenid der allgemeinen Formel IX ist Chloracetylchlorid, als Säureanhydrid der allgemeinen Formel X ist Chloressigsäureanhydrid bevorzugt. Als Lösungsmittel seien beispielsweise genannt aromatische Kohlenwasserstoffe, wie Toluol, Xylol oder Chlorbenzol ; offenkettige oder cyclische Ether, wie Diisopropylether oder Dioxan ; chlorierte Kohlenwasserstoffe, wie Dichlorethan, andere Lösungsmittel, wie Pyridin, Acetonitril oder Dimethylformamid.

Als Hilfsbasen seien z. B. tertiäre organische Basen, wie Triethylamin und Ethyldiisopropylamin, oder Pyridin ; oder anorganische Basen, wie wasserfreie Alkalimetall- oder Erdalkalimetallcarbonate oder/hydrogencarbonate oder Erdalkalimetalloxide genannt. Als Acylierungskatalysatoren kommen beispielsweise in Frage Imidazol, Pyridin oder 4-Dimethylaminopyridin.

Bedeutet in einer Verbindung der allgemeinen Formel II Hal ein Chloratom, so kann es durch Umsetzung mit NaJ in Aceton oder Ethanol leicht gegen das reaktivere Iod ausgetauscht werden.

Soweit die Ausgangsmaterialien der allgemeinen Formel IV nicht der allgemeinen Formel Ia (R=H) entsprechen, sind sie bekannt (EP-A 0 039 519 ; EP-A 0.057.428 ; DE-C 1.179.943 und 1.204.680 ; F. Hunzicker u.a., Arzneim.-Forsch. 13, 324 [1963]).

Ausgangsverbindungen der allgemeinen Formel III, in der Z eine Methylengruppe ist und $R_1$, $R_2$ und $A_2$ die eingangs definierten Bedeutungen haben, sind bekannt oder lassen sich in Analogie zu bekannten Verfahren herstellen. So erhält man beispielsweise solche Verbindungen der allgemeinen Formel III, in der $A_2$ eine Methylengruppen ist, durch Umsetzung von 2-, 3- oder 4-(Chlormethyl)pyridin-hydrochlorid mit einem sekundären Amin der allgemeinen Formel XI,

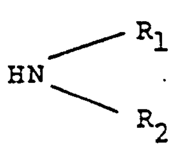

(XI)

in der $R_1$ und $R_2$ wie eingangs definiert sind (in Analogie zu A. Fischer u.a., Can. J. Chem. 56, 3059-3067 [1967]) und anschließende katalytische Hydrierung des erhaltenen tertiären Picolylamins, beispielsweise in ethanolisch-salzsaurer Lösung und unter Verwendung von Platin (IV)-oxid als Katalysator (siehe auch : F.F. Blicke u.a., J. Org. Chemistry 26, 3258 [1961]) oder in Eisessig in Gegenwart von Platin-(IV)-oxid (siehe auch : W.F. Minor u.a., J. Med. Pharm. Chem. 5, 96, 105ff [1962] und A.H. Sommers u.a., J. Amer. chem. Soc., 75, 57, 58ff. [1953]). Die unter die allgemeine Formel III fallenden 2-substituierten Pieperidine der allgemeinen Formel IIIa, in der $R_1$ und $R_2$ wie eingangs definiert sind,

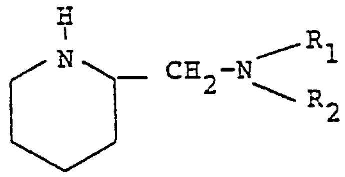

(IIIa)

können auch durch Umsetzung von 2-(Chlormethyl)piperidin-hydrochlorid (T.R. Norton, R.A. Seibert, A.A. Benson und F.W. Bergstrom, J. Amer. chem. Soc. 68, 1572-1576 [1946] bzw. M. Rinle und H.G. Liem, Archiv der Pharmazie 292, 165-169 [1959]) mit einem sekundären Amin der allgemeinen Formel XI bereitet werden. Nebenprodukte dieser Reaktion, die in Analogie zu Angaben von H. Biere und U. Redmann, Eur. J. Med. Chem. 11, 351-357 (1976), durchgeführt werden kann, sind in der Regel die gleichfalls unter die allgemeine Formel III fallenden 3-substituierten Hexahydroazepine der allgemeinen Formel III b.

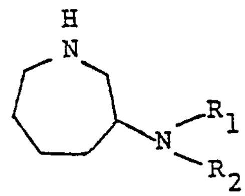

(IIIb)

IIIa und IIIb können nach üblichen Verfahren, beispielsweise durch fraktionierte Destillation oder durch fraktionierte Kristallisation ihrer Salze, etwa ihrer Dihydrochloride, leicht getrennt werden.

Die unter die allgemeine Formel III fallenden Ausgangsverbindungen der Formel IIIc,

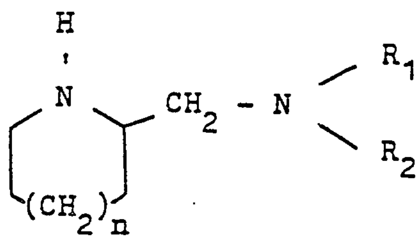

(IIIc)

in der $R_1$ und $R_2$ die eingangs angegebenen Bedeutungen besitzen und n die Werte 1 und 2 annehmen kann, erhält man auch nach den Angaben von H.J. Schmid u.a., Helv. Chim. Acta 39, 607-618 (1956) oder in Analogie dazu.

Ausgangsverbindungen der allgemeinen Formel III, in der Z eine Methylengruppe und $A_2$ eine 1,2-Ethylengruppe bedeuten, $R_1$ und $R_2$ wie eingangs definiert sind und die Seitenkette in 2-Stellung des Piperidinringes steht, erhält man durch Addition von Aminen der allgemeinen Formel XI an 2-Vinylpyridin (siehe auch F.F. Blicke u.a., J. org. Chemistry 26, 3257-3260 [1961] ; W.F. Minor u.a., J. Med. Pharm. Chem. 5, 96-107 [1962] ; A.H. Sommers u.a., J. Amer. chem. Soc. 75, 57-60 [1953] und nachfolgende katalytische Hydrierung unter den vorstehend genannten Bedingungen.

Die unter die Diamine der allgemeinen Formel III fallenden 2-[(Dialkylamino)methyl]pyrrolidine, bei denen Z eine Einfachbindung bedeutet, lassen sich nach oder in Analogie zu T. Sone u.a., Chem. Pharm. Bull. (Tokyo) 21, 2331 [1973] durch Reduktion entsprechender Prolinamide mit Lithiumaluminiumhydrid erhalten. Ersetzt man in dieser Synthese Prolin durch Hexahydro-1H-azepin-2-carbonsäure (siehe auch H.T. Nagasawa u.a., J. Med. Chem. 14, 501 [1971]), so gelangt man zu den unter die allgemeine Formel III fallenden 2-substituierten Hexahydro-1H-azepinen, in denen Z eine Ethylengruppe und $A_2$ eine Methylengruppe darstellen und $R_1$ und $R_2$ die eingangs angegebenen Bedeutungen haben.

Die unter die allgemeine Formel III fallenden 3-[(Dialkylamino)methyl]piperidine lassen sich auch aus entsprechenen Nicotinsäureamiden nach V.M. Micovic u.a., J. org. Chem. 18, 1196 [1953] bzw. F. Haglid u.a., Acta. Chem. Scand. 17, 1741 [1963] herstellen. Hexahydronicotin erhält man aus Nicotin nach den Angaben von W.R. Harlan und R.M. Hixon, J. Amer. chem. Soc. 52, 3385-3388 (1930).

3-(Dialkylamino)-pyrrolidine, -piperidine und -hexahydro-1H-azepine (III : $R_1$ und $R_2$ wie eingangs angegeben ; Z eine Methylen- oder Ethylengruppe oder eine Einfachbindung ; $A_2$ eine Einfachbindung)

lassen sich nach den Angaben von H.R. Bürki u.a., Eur. J. Med. Chem. 13, 479-485 [1978] und Smithkline Corp., US-A 3.980.788 ; C.A. 85, P 182415z [1976] aus N-Benzyl-3-pyrrolidinon-, -3-piperidinon bzw. -hexahydro-1H-azepin-3-on oder in Analogie dazu herstellen.

Verbindungen der allgemeinen Formel III, in der Z ein Sauerstoffatom ist und der Rest

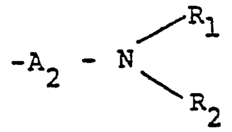 .

sich in 2-Stellung zur sekundären Aminogruppe befindet, sind aus 3-Oxomorpholin durch N-Benzylierung, Alkylierung mit Dialkylsulfaten, Kondensation mit Nitromethan, Abwandlung der Nitromethylen-Gruppierung durch Reduktion bzw. schonende katalytische Hydrierung, Abwandlung der primären Aminogruppe beispielsweise durch Eschweiler-Clarke-Methylierung oder Umsetzung mit geeigneten Alkylhalogeniden, Dialkylsulfaten oder α,ω-Dihalogenalkanen und schließlich hydrogenolytische Abspaltung der Schutzgruppe zugänglich.

Die Ausgangsverbindungen der allgemeinen Formel V, in der Nu eine Alkoxygruppe bedeutet, erhält man durch Umsetzung von Diaminen der allgemeinen Formel III mit Halogenessigsäureestern, β-Halogenpropionsäureestern bzw. Acrylestern, gegebenenfalls unter Verwendung zusätzlicher Hilfsbasen, z. B. Triethylamin, oder Katalysatoren, beispielsweise Triton B. Durch Verseifung der erhaltenen Ester, z. B. mit Barytlauge, erhält man die unter die allgemeine Formel V fallenden Carbonsäuren, die zur Herstellung von Derivaten mit anderen nucleofugen Gruppen dienen können.

Ein weiterer Gegenstand der Erfindung sind Arzneimittel, die ein oder mehrere kondensierte Diazepinone der allgemeinen Formel I bzw. deren physiologisch verträgliche Salze enthalten.

Die Verbindungen der allgemeinen Formel I lassen sich hierzu in an sich bekannter Weise in die üblichen pharmazeutischen Zubereitungsformen, z. B. in Lösungen, Suppositorien, Tabletten, Dragées, Kapseln oder Teezubereitungen einarbeiten. Die Tagesdosis liegt im allgemeinen zwischen 0,02 und 5 mg, vorzugsweise 0,02 und 2,5 mg, insbesondere 0,05 und 1,0 mg/kg Körpergewicht, die gegebenenfalls in Form mehrerer, vorzugsweise 1 bis 3 Einzelgaben, zur Erzielung der gewünschten Ergebnisse verabreicht wird.

Die basisch substituierten kondensierten Diazepinone der allgemeinen Formel I und ihre Säureadditionssalze besitzen wertvolle Eigenschaften ; insbesondere besitzen sie günstige Effekte auf die Herzfrequenz und sind angesichts fehlender magensäuresekretionshemmender, salivationshemmender und mydriatischer Einflüsse als vagale Schrittmacher zur Behandlung von Bradycardien und Bradyarrhythmien in der Human- und auch der Veterinärmedizin geeignet ; ein Teil der Verbindungen zeigt auch spasmolytische Eigenschaften auf periphere Organe, insbesondere Colon und Blase.

Darüber hinaus zeigt ein Teil der Verbindungen an der Maus in der Versuchsanordnung nach W.W. Duke, J. Amer. Med. Ass. 15, 1185 (1910) im Dosisbereich zwischen 0,1 und 10 mg/kg p.o. Blutungszeitverlängernde Eigenschaften.

Eine günstige Relation zwischen tachycarden Wirkungen einerseits und den bei Therapeutika mit anticholinerger Wirkkomponente auftretenden unerwünschten Wirkungen auf die Pupillenweite und Tränen-, Speichel- und Magensäuresektretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

A. Untersuchung auf funktionelle Selektivität der antimuscarinischen Wirkung

Substanzen mit antimuscarinischen Eigenschaften inhibieren die Wirkungen von exogen zugeführten Agonisten oder von Acetylcholin, das aus cholinergen Nervenendigungen freigesetzt wird. Im folgenden wird eine Beschreibung von Methoden wiedergegeben, die zur Erfassung von cardioselektiven Antimuscarinica geeignet sind.

« In vitro » Organpräparationen

Dissoziationskonstanten ($K_B$-Werte) wurden « in vitro » am Ileum und elektrisch stimulierten Vorhof des Meerschweinchens ermittelt. Das Ileum wurde entnommen und im Organbad in Tyrode-Lösung inkubiert. Kontraktionen wurden derart durch steigende Konzentrationen von Bethanechol (B) hervorgerufen, daß volle Konzentrations-Wirkungskurven aufgenommen werden konnten. Danach wurde B ausgewaschen, die zu untersuchende Substanz beigefügt und 2 Minuten lang in Kontakt gelassen, und wiederum eine Konzentrations-Wirkungskurve mit B aufgenommen.

Aus dem Dosisverhältnis (DR), das ist das Ausmaß der Verschiebung der Konzentrations-Wirkungskurve, wurde die Dissoziationskonstante nach Arulakshana und Schlid (Brit. J. Pharmacol. 14, 48, 1959) berechnet.

Am isolierten, elektrisch stimulierten linken Vorhof reduzierte B konzentrationsabhängig die

Kontraktionskraft. Durch Zugabe eines Antimuscarinicums wurde dieser Effekt wieder aufgehoben. Dissoziationskonstanten für die muscarinischen Rezeptoren des Vorhofes wurden auf die gleiche Art und Weise wie oben beschrieben gewonnen. Der Vergleich der in beiden Geweben ermittelten Dissoziationskonstanten erlaubte die Identifizierung von cardioselektiven Substanzen.

« In vivo » Methoden

Die angewandten Methoden hatten zum Ziel, die Selektivität der antimuscarinischen Wirkung zu bestätigen. Jene Substanzen, die auf der Basis von « in vitro » Untersuchungen ausgewählt worden waren, wurden auf ihre

1. Tachycarde Wirkung am wachen Hund,
2. Magensäuresekretionshemmende Wirkung an der Pylorus-ligierten Ratte und
3. Mydriatische Wirkung an der Ratte

untersucht.

1. Herzfrequenz steigernde Wirkung am wachen Hund

Die Substanzen wurden intravenös injiziert und die Herzfrequenz mit Hilfe eines Tachygraphen gemessen. Nach einem Kontrollzeitraum wurden steigende Dosen der Verbindung appliziert, um die Herzfrequenz zu erhöhen. Es wurde jeweils dann die nächste Dosis injiziert, wenn der Effekt der vorangegangenen nicht mehr sichtbar war. Die Dosis einer Substanz, die eine Erhöhung um 50 Schläge/Minute ($ED_{50}$) bewirkte, wurde graphisch ermittelt. Jede Substanz wurde an 3 bis 5 Hunden untersucht.

2. Sekretionshemmung an Ratten

Die angewandte Methode wurde von Shay et al. (Gastroenterology, 26, 906, 1954) beschrieben. Steigende Dosen der Substanz wurden 5 Minuten vor der Pylorus-Ligatur in männliche Wistar-Ratten intravenös injiziert (10 Ratten/Gruppe). Die Ratten wurden 2 Stunden später getötet und sowohl das Magensaftvolumen als auch der « Acid Output » durch Titration bestimmt. Die Dosis der Substanz, die um 50 % entweder das Volumen oder den « Acid Output » verminderte, wurde graphisch ermittelt.

3. Mydriatische Wirkung an Ratten

Die Mydriasis wurde durch Messung der Zunahme der Pupillenweite nach intravenöser Injektion der zu untersuchenden Substanz bestimmt. Die Messung der Pupillenweite erfolgte mit einem Mikroskop. Die Messungen wurden vor und zu verschiedenen Zeiten (15, 45 und 75 Minuten) nach der Injektion unterschiedlicher Dosen der Substanz vorgenommen. Die Resultate wurden als $ED_{200}$ ausgedrückt. Das ist jene Dosis, die eine Verdoppelung des Pupillendurchmessers, bezogen auf den Basalwert, bewirkte. Der maximale Effekt war gewöhnlich zwischen 15 und 45 Minuten nach intravenöser Gabe zu beobachten.

B. Bindungsstudien an muscarinischen Rezeptoren :
Bestimmung des $IC_{50}$-Wertes

Als Organspender dienten männliche Sprague-Dawley-Ratten mit 180-220 g Körpergewicht. Nach Entnahme von Herz, Magen und Großhirnrinde wurden alle weiteren Schritte in eiskaltem Hepes-HCl-Puffer (pH 7,4 ; 100 m molar NaCl, 10 m molar $MgCl_2$) vorgenommen. Das Gesamtherz wurde mit einer Schere zerkleinert. Alle Organe wurden abschließend in einem Potter homogenisiert.

Für den Bindungstest wurden die Organhomogenate in folgender Weise verdünnt :

| | |
|---|---|
| Gesamtherz | 1 : 250 |
| Großhirnrinde | 1 : 3000 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30 °C. Die Inkubationsdauer betrug 45 Minuten. Als Radioligand wurde 0,3 n molar $^3$H-N-Methylscopolamin ($^3$H-NMS) verwendet. Nach Beendigung der Inkubation durch Zentrifugation bei 14 000 g wurde die Radioaktivität im Pellet bestimmt. Sie repräsentiert die Summe von spezifischer und unspezifischer Bindung von $^3$H-NMS. Der Anteil der unspezifischen Bindung wurde definiert als jene Radioaktivität, die in Anwesenheit von 1 $\mu$ molar Chinuclidinylbenzilat gebunden wurde. Es wurden immer Vierfachbestimmungen vorgenommen. Die $IC_{50}$-Werte der nichtmarkierten Testsubstanzen wurden graphisch bestimmt. Sie repräsentieren jene Konzentration der Testsubstanz, bei welcher die spezifische Bindung von $^3$H-NMS an die muscarinischen Rezeptoren in den verschiedenen Organen um 50 % gehemmt wurde.

Nach den vorstehenden Angaben wurden biespielsweise die folgenden Verbindungen untersucht :

A = 11-[[2-[(Diethylamino)methyl]-1-piperidinyl]acetyl]-5,11-dihydro-6H-pyrido [2,3-b] [1,4] benzodiazepin-6-on

B = 5,11-Dihydro-11-[(4-methyl-1-piperazinyl) acetyl]-6H-pyrido [2,3-b] [1,4] benzodiazepin-6-on (Pirenzepin)

C = Atropin

D = 5,11-Dihydro-11-[[2-(dimethylamino)methyl]-1-piperidinyl]acetyl]-6H-pyrido [2,3-b] [1,4] benzodiazepin-6-on

E = 5,11-Dihydro-11-[[2-[2-(dimethylamino)ethyl]-1-piperidinyl]acetyl]-6H-pyrido [2,3-b] [1,4] benzodiazepin-6-on

F = 5-[[2-[(Diethylamino)methyl]-1-piperidinyl]acetyl]-5,10-dihydro-11H-dibenzo [b,e] [1,4] diazepin-11-on

G = 4-[[2-[(Diethylamino)methyl]-1-piperidinyl]acetyl]-4,9-dihydro-10H-thieno [3,4-b] [1,5] benzodiazepin-10-on

H = (S)-11-[[2-[(Diethylamino)methyl]-1-pyrrolidinyl]acetyl]-5,11-dihydro-6H-pyrido [2,3-b] [1,4] benzodiazepin-6-on

I = (S)-5-[[2-[(Diethylamino)methyl]-1-pyrrolidinyl]acetyl]-5,10-dihydro-11H-dibenzo [b,e] [1,4] diazepin-11-on

K = trans-5,11-Dihydro-11-[[2-[[(4-hydroxycyclohexyl)-(methyl)amino]methyl]-1-piperidinyl]acetyl]-6H-pyrido [2,3-b] [1,4] benzodiazepin-6-on

L = 11-[[3-[[(Cyclohexyl) (methyl)amino]methyl]-1-piperidinyl]acetyl]-5,11-dihydro-6H-pyrido [2,3-b] [1,4] benzodiazepin-6-on.

M = 5-[[3-[(Diethylamino)methyl]-1-piperidinyl]acetyl]-5,10-dihydro-11H-dibenzo [b,e] [1,4] diazepin-11-on

N = 5-[[2-[[(Cyclohexyl)methyl)amino]methyl]-1-piperidinyl]acetyl]-5,10-dihydro-11H-dibenzo [b,e] [1,4] diazepin-11-on

O = 5-[[3-[[(Cyclohexyl) (methyl)amino]methyl]-1-piperidinyl]-acetyl]-5,10-dihydro-11H-dibenzo [b,e] [1,4] diazepin-11-on

P = 5,10-Dihydro-5-[[3-(1-methyl-2-pyrrolidinyl)-1-piperidinyl]acetyl]-11H-dibenzo [b,e] [1,4] diazepin-11-on

Q = 11-[[2-[(Butylmethylamino) methyl]-1-piperidinyl] acetyl]-5,10-dihydro-11H-dibenzo [b,e] [1,4] diazepin-11-on

(Siehe Tabellen Seite 14 f.)

Tabelle I
Ergebnisse

| Substanz | Rezeptor-Bindungs-Tests $IC_{50}$ [$nMl^{-1}$] Cortex | Herz |
|---|---|---|
| A | 1200<br>3000 | 140<br>150 |
| B | 100 | 1500 |
| C | 2 | 4 |
| D | 1500 | 150<br>130 |
| E | 1300 | 170 |
| F | 750<br>1000 | 150<br>100 |
| G | 2200 | 220 |
| H | 4500 | 400 |
| I | 800 | 90 |
| K | 1100 | 70 |
| L | 85 | 19 |
| M | 100 | 35 |
| N | 50 | 13 |
| O | 20 | 2,8 |
| P | 120 | 17 |
| Q | 100 | 18 |

Tabelle II
Ergebnisse

| Substanz | Tachycardie (Hund) $ED_{50}$ [µg/kg] i.v. | Sekretionshemmung (Shay-Ratte) $ED_{50}$ [µg/kg] i.v. | Mydriasis (Ratte) $ED_{50}$ [µg/kg] i.v. |
|---|---|---|---|
| A | 103 | > 10000 | > 3000 |
| B | 468 | 440 | 408 |
| C | 7,7 | 31 | 4 |

Die Angaben in der vorstehenden Tabelle I beweisen, daß die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus der Großhirnrinde gegenüber solchen aus der glatten Muskulatur es Herzens.

Aus den pharmakologischen Daten der vorstehenden Tabelle II ergibt sich — in völliger Übereinstimmung mit den Rezeptor-Bindungs-Studien —, daß die Herzfrequenz durch die genannten Verbindungen bereits bei Dosierungen gesteigert wird, bei denen noch keine Einschränkung der Magensäuresekretion und keine Mydriasis beobachtet werden.

Ferner sind die erfindungsgemäß hergestellten Verbindungen gut verträglich, so konnten selbst bei den höchsten applizierten Dosen bei den pharmakologischen Untersuchungen keine toxischen Nebenwirkungen beobachtet werden.

Die nachfolgenden Beispiele sollen die Erfindung näher erläutern :

« Fp. » bedeutet « Schmelzpunkt », « Z. » bedeutet « Zersetzung ». Für alle Verbindungen liegen befriedigende Elementaranalysen, IR-, UV-, $^1$H-NMR-, häufig auch Massenspektren vor.

Herstellung der Ausgangsmaterialien

## Beispiel A

2-[(1-Pyrrolidinyl)methyl]pyridin

Unter Rühren und äußerer Kühlung mit Eis tropfte man zu 500 ml (426 g ; 5,99 Mol) Pyrrolidin die Lösung von 100 g (0,61 Mol) 2-(Chlormethyl)pyridin-hydrochlorid in 600 ml 95 proz. Ethanol und erhitzte die resultierende Mischung 1 Stunde lang auf 90 °C. Die erkaltete Mischung wurde abgenutscht, der Filterrückstand mit Ether gründlich ausgewaschen, das Filtrat im Vakuum eingedampft und der Rückstand mit 40 proz. Kalilauge stark alkalisch gestellt. Man extrahierte erschöpfend mit Ether, befreite die erhaltenen Auszüge vom Lösungsmittel und destillierte den verbliebenen Rückstand im Vakuum. Man erhielt 93,0 g (94 % der Theorie) der gewünschten Verbindung. Siedepunkt 2,53 kPa (19 mm Hg) 118-121 °C.

Entsprechend wurden hergestellt :

2-[(Dimethylamino)methyl]pyridin vom Siedepunkt 0,04 kPa (0,3 mm Hg) 30 °C ;

2-[(Diethylamino)methyl]pyridin vom Siedepunkt 2,66 kPa (20 mm Hg) 104-106 °C ;

2-[(4-Morpholinyl)methyl]pyridin vom Siedepunkt 0,53 kPa (4 mm Hg) 106-107 °C ;

2-[[(Cyclohexyl) (methyl)amino]methyl]-pyridin vom Siedepunkt 0,53 kPa (4 mm Hg) 108-110 °C ;

2-[(1-Piperidinyl)methyl]pyridin vom Siedepunkt 2,66 kPa (20 mm Hg) 125-130 °C ;

2-[(4-Methyl-1-piperazinyl)methyl]pyridin vom Siedepunkt 2,4 kPa (18 mm Hg) 148-153 °C ; Schmelzpunkt des Monohydrochlorids 175-176 °C ;

trans-2-[[(4-Hydroxycyclohexyl) (methyl) amino]methyl]-pyridin vom $R_{F0,58}$ (Polygram SIL $G/UV_{254}$ [Macherey-Nagel] ;

Fließmittel : Methylenchlorid/Methanol/Cyclohexan/konz. Ammoniak = 68 : 15 : 15 : 2).

3-[[(Cyclohexyl) (methyl)amino]methyl]pyridin, Siedepunkt 1,35 kPa (10 mm Hg) 148-153 °C ;

3-[(Dimethylamino)methyl]pyridin, Siedepunkt 1,33 kPa (10 mm Hg) 82 °C ;

3-[(1-Pyrrolidinyl)methyl]pyridin, Siedepunkt 1,47 kPa (11 mm Hg) 122-125 °C ;

3-[(1-Piperidinyl)methyl]pyridin, Siedepunkt 1,47 kPa (11 mm Hg) 128-130 °C ;

3-[(Diethylamino)methyl]pyridin, Siedepunkt 1,87 kPa (14 mm Hg) 108-109 °C ;

2-[[(2-Hydroxyethyl) (methyl)amino]methyl]pyridin, Siedepunkt 2,4 kPa (18 mm Hg) 162 °C ;

2-(Butylmethylamino)methyl]pyridin, Siedepunkt 2,66 kPa (20 mm Hg) 109-110 °C ;

2-[(Ethylmethylamino)methyl]pyridin, Siedepunkt 2,26 kPa (17 mm Hg) 86 °C.

## Beispiel B

2-[(4-Morpholinyl)methyl]piperidin

94,0 g (0,53 Mol) 2-[(4-Morpholinyl)methyl]pyridin wurden in 600 ml Eisessig gelöst und in Gegenwart von 10,0 g Platin-(IV)-oxid als Katalysator 2 Stunden bei einem Wasserstoffdruck von 3 bar und einer Temperatur von 50 °C hydriert. Nach dem Erkalten wurde vom Katalysator abfiltriert, das Filtrat vom Lösungsmittel befreit und der Rückstand im Vakuum destilliert. Man erhielt 51,0 g (52 % der Theorie) der gewünschten Verbindung in Form einer farblosen Flüssigkeit vom Siedepunkt 0,53 kPa (4 mm Hg) 91-92 °C.

Entsprechend wurden hergestellt :

2-[(Dimethylamino)methyl]piperidin, Siedepunkt 2,00 kPa (15 mm Hg) 71-75 °C ;

2-[(Diethylamino)methyl]piperidin, Siedepunkt 2,26 kPa (17 mm Hg) 88-94 °C ;

2-[2-(Dimethylamino)ethyl]piperidin, Siedepunkt 3,73 kPa (28 mm Hg) 102-106 °C ;

2-[2-(Diethylamino)ethyl]piperidin, Siedepunkt 2,66 kPa (20 mm Hg) 118-120 °C ;

2-[(1-Pyrrolidinyl)methyl]piperidin, Siedepunkt 2,66 kPa (20 mm Hg) 115 °C ;

2-[[(Cyclohexyl) (methyl)amino]methyl]piperidin, Siedepunkt 0,53 kPa (4 mm Hg) 103-108 °C ;
2-[(1-Piperidinyl)methyl]piperidin, Siedepunkt 2,4 kPa (18 mm Hg) 115-125 °C ;
2-[(4-Methyl-1-piperazinyl)methyl]piperidin, Siedepunkt 2,4 kPa (18 mm Hg) 142-145 °C ;
$n_D^{20}$ 1,4959 ;
trans-2-[[(4-Hydroxycyclohexyl) (methyl)amino]lmethyl]-piperidin, $R_F$ 0,55
Polygram SIL G/$UV_{254}$ [Macherey-Nagel] ; Fließmittel : Dichlormethan+Methanol+Cyclohexan +konz. Ammoniak=68+15+15+2).
Fp. 58-60 °C.
2-[[(2-Hydroxyethyl) (methyl)amino]methyl]piperidin, Siedepunkt 2,4 kPa (18 mm Hg) 144-146 °C ;
2-[(Butylmethylamino)methyl]piperidin, Siedepunkt 2,66 kPa (20 mm Hg) 115 °C ;
2-[(Ethylmethylamino)methyl]piperidin, Siedepunkt 2,4 kPa (18 mm Hg) 79-80 °C ;
3-[[(Cyclohexyl) (methyl)amino]methyl]piperidin, Siedepunkt 1,47 kPa (11 mm Hg) 139-153 °C ;
3-[(Dimethylamino)methyl]piperidin, Siedepunkt 1,60 kPA (12 mm Hg) 74-75 °C ;
3-[(1-Pyrrolidinyl)methyl]piperidin, Siedepunkt 1,73 kPa (13 mm Hg) 123-124 °C ;
3-[(1-Piperidinyl)methyl]piperidin, Siedepunkt 3,07 kPa (23 mm Hg) 141-145 °C ;
3-[(Diethylamino)methyl]piperidin, Siedepunkt 1,60 kPa (12 mm Hg) 106-108 °C ;

### Beispiel C

(S)-2-[(1-Pyrrolidinyl)methyl]pyrrolidin

a.) (S)-1-[[1-[[(Phenylmethyl)oxy]carbonyl]-2-pyrrolidinyl]carbonyl]-pyrrolidin

Das Gemisch aus 30,0 g (0,12 Mol) N-Carbobenzoxy-L-prolin, 200 ml wasserfreiem Tetrahydrofuran und 20 ml (0,257 Mol) Thionylchlorid wurde 2 Stunden unter Rückfluß gekocht und anschließend im Wasserstrahlvakuum eingedampft. Der ölige Rückstand wurde in 200 ml wasserfreiem Tetrahydrofuran aufgenommen und unter äußerer Kühlung mit Wasser tropfenweise mit der Lösung von 41,8 ml (0,5 Mol) Pyrrolidin in 200 ml Tetrahydrofuran versetzt. Nach 2-stündigem Kochen wurde das Lösungsmittel abgedampft, der Rückstand mit überschüssiger, gesättigter, wäßriger Kaliumcarbonat-Lösung versetzt und mit Essigsäureethylester erschöpfend extrahiert. Die vereinigten Extrakte wurden über Natriumsulfat getrocknet und eingedampft, der verbleibende farblose Rückstand aus Aceton/Petrolether (3 : 1 v/v) umkristallisiert. Man erhielt 26,0 g (72 % der Theorie) an farblosen Kristallen vom Fp. 138 °C.

Entsprechend wurden hergestellt :
(S)-N,N-Diethyl-1-[[(phenylmethyl)oxy]carbonyl]-prolinamid, gelbes Öl ;
(S)-4-Methyl-1-[[(phenylmethyl)oxy]carbonyl]-2-pyrrolidinyl]-carbonyl]-piperazin, Fp. 95-96 °C.

b.) (S)-1-[(2-Pyrrolidinyl)carbonyl]-pyrrolidin

18,6 g (0,0615 Mol) (S)-1-[[1-[[(Phenylmethyl)oxy]carbonyl]-2-pyrrolidinyl]carbonyl]-pyrrolidin wurden in 100 ml Ethanol gelöst und nach Zugabe von 1,0 g 5proz. Palladium/Tierkohle-Katalysator bei Zimmertemperatur und 1 bar Wasserstoffdruck bis zur Beendigung der Umsetzung hydriert. Die Mischung wurde filtriert, das Filtrat im Vakuum eingedampft. Der Rückstand wurde im Vakuum destilliert. Man erhielt 8,4 g (81 % der Theorie) eines farblosen Öls vom Siedepunkt 0,53 kPa (4 mm Hg) 134-136 °C.

Entsprechend wurden hergestellt :
(S)-N,N-Diethyl-prolinamid, Siedepunkt 0,67 kPa (5 mm Hg) 107-108 °C ;
(S)-4-Methyl-1-[(2-pyrrolidinyl)carbonyl[-piperazin, Öl.

c.) (S)-2-[(1-Pyrrolidinyl)methyl]pyrrolidin

Eine wasserfreie Lösung von 21,87 g (0,13 Mol) (S)-1-[(2-Pyrrolidinyl)carbonyl]-pyrrolidin in 100 ml wasserfreiem Tetrahydrofuran wurde unter Rühren und äußerer Kühlung mit Eis zu einer Suspension von 7,4 g (0,2 Mol) Lithiumaluminiumhydrid in 200 ml trockenem Tetrahydrofuran zugetropft. Anschließend kochte man 18 Stunden unter Rückfluß, ließ erkalten und zersetzte das überschüssige komplexe Hydrid durch Zugabe von 7 ml Wasser, dann 7 ml 15proz. Natronlauge und schließlich 15 ml Wasser. Nach Zugabe von 100 ml Tetrahydrofuran kochte man noch 30 Minuten unter Rückfluß, ließ erkalten und trocknete die Mischung durch Zugabe von reichlich wasserfreiem Kaliumcarbonat. Man filtrierte, engte das Filtrat im Vakuum ein und destillierte den öligen Rückstand bei vermindertem Druck. Man erhielt 16,6 g (83 % der Theorie) eines farblosen Öls vom Siedepunkt 1,60 kPa (12 mm Hg) 95-98 °C. $[\alpha]_D^{20}$ + 4,45° (Ethanol).

Entsprechend wurden hergestellt :
(S)-2-[(Diethylamino)methyl]pyrrolidin, Siedepunkt 3,4 kPa (30 mm Hg)
100-105 °C, $[\alpha]_D^{20}$ + 11,3° (Ethanol)
[Lit. : $[\alpha]_D^{20}$ + 13,4° (Ethanol)]

(S)-4-Methyl-1-[(2-pyrrolidinyl)methyl]piperazin, Siedepunkt 1,60 kPa (12 mm Hg) 125 °C, $[\alpha]_D^{20}$ + 10,1°

(S)-4-[(2-Pyrrolidinyl)methyl]morpholin, Siedepunkt 1,60 kPa (12 mm Hg) 123 °C

## Beispiel D

2-[(Diethylamino)methyl]piperidin und 3-(Diethylamino)-hexahydro-1H-azepin

a.) 2-[(Diethylamino)methyl]piperidin, Rohprodukt

Die Mischung von 418 g (2,457 Mol) 2-(Chlormethyl)piperidin-hydrochlorid (M. Rink und H.G. Liem, Archiv der Pharmazie 292, 165-169 [1959]), 2,487 l (24,17 Mol) Diethylamin, 1,2 l Methanol und 4,13 g (0,0276 Mol) Natriumjodid wurde bis zur vollständigen Umsetzung (ca. 17 Stunden) unter Rückfluß gekocht. Man dampfte im Vakuum ein, stellte den Rückstand mit der Lösung von 150 g (2,673 Mol) Kaliumhydroxid in 150 ml Wasser alkalisch und extrahierte die erhaltene Mischung viermal mit je 1 l Diethylether. Die vereinigten Etherauszüge wurden über Natriumsulfat getrocknet, vom Lösungsmittel und überschüssigen Diethylamin befreit und der Rückstand im Wasserstrahlvakuum fraktioniert destilliert. Man erhielt 218 g (52 % der Theorie) eines farblosen Öls vom Sdp 20 mm Hg 92-94 °C, das nach dünnschichtchromatographischer Untersuchung (Kieselgel-Fertigplatten 60 F 254 Merck, Fließmittel: Dichlormethan + Methanol + Cyclohexan + konz. Ammoniak = 68 + 15 + 15 + 2 ; Detektion : Ansprühen mit 1 proz. wäss. $KMnO_4$-Lösung ; RF = 0,47) noch zu ca. 10 % durch einen Begleiter mit $R_F$ 0,38 verunreinigt war. Die bei Sdp 20 mm Hg 94-100 °C übergehenden Fraktionen (zusammen 55 g) ergaben nach Redestillation weitere 41,3 g (9,9 % der Theorie) eines Öls vom Sdp 2,67 kPa (20 mm Hg) 95-97 °C, das den Begleiter nur noch in einer Konzentratrion von ca. 3 % enthielt. Gesamtausbeute : 259,3 g (62 % der Theorie).

Beide Fraktionen wurden vereinigt und zwecks Abtrennung des störenden 3-(Diethylamino)-hexahydro-1H-azepins ins Dihydrochlorid übergeführt.

b.) 2-[(Diethylamino)methyl]piperidin-dihydrochlorid

Zu der Lösung des in Stufe a.) erhaltenen rohen 2-[(Diethylamino)methyl]piperidins (259 g = 1,521 Mol) in 648 ml 2-Propanol ließ man unter Rühren die Lösung von 122,4 g (3,357 Mol) gasförmigem Chlorwasserstoff in 687 ml 2-Propanol zulaufen. Dabei erwärmte sich die Mischung und färbte sich dunkel. Man ließ über Nacht bei Zimmertemperatur stehen, nutschte den Niederschlag scharf ab und wusch ihn achtmal mit je 15 ml kaltem 2-Propanol gründlich durch. Das noch lösungsmittelfeuchte Salz wurde anschließend aus 400 ml heißem 2-Propanol in üblicher Weise umkristallisiert, wonach das erhaltene Kristallisat abermals durch achtmaliges Waschen mit je 15 ml 2-Propanol von anhaftenden Mutterlaugen sorgfältig befreift wurde. Das erhaltene farblose Produkt wurde im Umlufttrockenschrank bei 60 °C getrocknet. Man erhielt 284 g (76,8 % der Theorie), des gesuchten Dihydrochlorids vom Fp. 158-160 °C. Die Gesamtausbeute der Stufen a) und b) betrug 47,5 % der Theorie.

Bei DC-Untersuchung (wie in Stufe a)) waren bei einem Auftrag von 200 γ Verunreinigungen nicht erkennbar ; die gaschromatographische Untersuchung ergab, daß das Produkt noch 0,1 % des Begleiters 3-(Diäthylamino)-hexahydro-1H-azepin bzw. seines Salzes enthielt.

c.) 3-(Diethylamino)-hexahydro-1H-azepin

Die Mutterlaugen der vorstehenden Umkristallisationen wurden vereinigt und im Vakuum eingedampft. Der Rückstand wurde alkalisch gestellt und in üblicher Weise aufgearbeitet. Das so erhaltene Basengemisch wurde mit den Rückständen der unter a) beschriebenen Destillation vereinigt. Durch mehrfache sorgfältige Destillation mit einer 90 cm langen, mit Keramik-Füllkörpern beschickten Kolonne wurde eine Fraktion von Sdp 2,67 kPa (20 mm Hg) 108-109 °C erhalten. Die nach gaschromatographischer und spektroskopischer Untersuchung zu mindestens 98,5 % aus 3-(Diethylamino)-hexahydro-1H-azepin-bestand und höchstens noch 0,5 % 2-[(Diethylamino)methyl]piperidin enthielt.

## Beispiel E

2-[(Diethylamino)methyl]-hexahydro-1H-azepin

a.) 7-[(Diethylamino)methyl]-hexahydro-2H-azepin-2-on-hydrochlorid

Zu der Lösung von 22,0 g (0,1 Mol) 2-[(Diethylamino)methyl]-cyclohexanon in 200 ml konzentrierter Salzsäure tropfte man unter Einhaltung einer Reaktionstemperatur von — 10 bis — 5 °C die Lösung von 10,0 g (0,154 Mol) Natriumazid in möglichst wenig Wasser. Nach Entfernung der Kühlung rührte man die Mischung 48 Stunden bei Zimmertemperatur. Nach erneuter Kühlung auf — 10 bis — 5 °C wurden

nochmals 5,0 g (0,077 Mol) Natriumazid in gleicher Weise wie oben zugegeben, wonach abermals 24 Stunden bei Raumtemperatur gerührt wurde. Dann wurde die Reaktionsmischung im Wasserstrahlvakuum eingedampft, der sirupöse Rückstand in 10 ml Wasser aufgenommen, mit reichlich wasserfreiem Natriumcarbonat versetzt und gründlich mit 200 ml Essigsäureethylester verrührt. Die organische Phase hinterließ nach dem Entfernen des Lösungsmittels einen öligen Rückstand, der sich durch Behandlung mit etherischer Chlorwasserstoff-Lösung in ein Hydrochlorid überführen ließ.

Fp. 171 °C (Essigsäureethylester/Methanol 3 : 1 v/v).

Ausbeute : 10,1 g (43 % der Theorie).

b.) 2-[(Diethylamino)methyl]-hexahydro-1H-azepin

In die Suspension von 1,9 g (0,05 Mol) Lithiumaluminiumhydrid in 500 ml wasserfreiem Tetrahydrofuran trug man unter äußerer Kühlung 10,0 g (0,043 Mol) 7-[(Diethylamino)methyl]-hexahydro-2H-azepin-2-on-hydrochlorid portionsweise ein und kochte nach Beendigung der anfangs heftigen Reaktion 1 Stunde unter Rückfluß. Das überschüssige Lithiumaluminiumhydrid wurde durch tropfenweise Zugabe von 20proz. Natronlauge zersetzt, die Tetrahydrofuran-Phase vom entstandenen Niederschlag abgetrennt und im Vakuum eingeengt. Der verbleibende ölige Rückstand wurde abschließend im Wasserstrahlvakuum über Ätzkali destilliert. Man erhielt 4,7 g (59 % der Theorie) eines farblosen Öls vom Siedepunkt 1,60 kPa (12 mm Hg) 102-105 °C.

Herstellung der Endprodukte :

Beispiel 1

3-Chlor-4-[[2-[(dimethylamino)methyl]-1-piperidinyl]acetyl]-1-methyl-1,4,9,10-tetrahydropyrrolo[3,2-b][1,5]benzodiazepin-10-on

a.) 2,2,3-Trichlorpropionitril

Unter Rühren und Belichtung mit einer 300 W-Tageslichtlampe wurden in 300 g (3,351 Mol) 3-Chlorpropionitril ($n_D^{20}$ 1,4383) 426 g (6,008 Mol) über konz. Schwefelsäure getrocknetes Chlor eingeleitet. Die Reaktionstemperatur wurde dabei auf 70 bis 80 °C einreguliert. Nach Beendigung der Reaktion ließ man erkalten und spülte mit Stickstoff. Das erhaltene Produkt wurde im Wasserstrahlvakuum an einer Vigreux-Kolonne fraktioniert destilliert. Die nach spektroskopischer Untersuchung etwa 90proz. Fraktion vom Siedepunkt 2,4 kPa (18 mm Hg) 53-60 °C ($n_D^{20}$ 1,4678) wurde ohne weitere Reinigung für die folgende Umsetzung verwendet. Ausbeute : 404,9 g (85 % der Theorie).

b.) Diastereomerengemisch von 2-Chlor-3-[[[(ethoxy)carbonyl]-methyl] (methyl)amino]-2-propennitril

Zu der Lösung von 79,0 g (0,499 Mol) 2,2,3-Trichlorpropionitril in 200 ml Tetrahydrofuran tropfte man innerhalb von 15 Minuten die Mischung von 58,5 g (0,499 Mol) Sarcosinethylester, 200 ml Tetrahydrofuran und 101,2 g (1 Mol) Triethylamin. Innerhalb von 1 Stunde stieg die Temperatur der Mischung von selbst auf 30 °C. Zur Vervollständigung der Reaktion wurde anschließend 3 Stunden unter Rückfluß gekocht. Man ließ erkalten, rührte den Reaktionsansatz in 4 l Diethylether ein, filtrierte vom ausgefallenen Triethylamin-hydrochlorid ab und engte die etherische Lösung ein. Den Rückstand destillierte man im Feinvakuum.

Ausbeute : 97,1 g (96 % der Theorie) eines farblosen Öls vom Siedepunkt 0,04 kPa (0,3 mm Hg) 120-130 °C, das die beiden Isomeren laut $^1$H-NMR-spektroskopischer Untersuchung im ungefähren Mengenverhältnis 1 : 1 enthielt. Aus diesem Öl kristallisierte gelegentlich ein Isomeres, vermutlich das mit der (E)-Konformation, aus : Fp. 57 °C.

c.) 3-Amino-4-chlor-1-methyl-1H-pyrrol-2-carbonsäureethylester

0,5 g (0,0044 Mol) Kalium-tert.butanolat wurden in 300 ml wasserfreiem Toluol vorgelegt. Zu der erhaltenen Suspension tropfte man bei Zimmertemperatur die Lösung von 58,0 g (0,286 Mol) (E)-2-Chlor-3-[[[(ethoxy)carbonyl]methyl]-(methyl)amino]-2-propennitril (Fp. 57 °C) in 200 ml wasserfreiem Toluol und ließ 30 Minuten nachrühren. Die Mischung wurde in 1 l Eiswasser eingegossen, mit Essigsäure neutralisiert und anschließend mit Dichlormethan erschöpfend extrahiert. Die vereinigten Extrakte wurden über Natriumsulfat getrocknet und im Vakuum eingedampft. Der verbleibende Rückstand wurde aus Diisopropylether/Petrolether (1 : 1 v/v) umkristallisiert und ergab 50,0 g (86 % der Theorie) an farblosen Kristallen vom Fp. 48 °C.

d.) 4-Chlor-1-methyl-3-[(2-nitrophenyl)amino]-1H-pyrrol-2-carbonsäureethylester

Unter äußerer Kühlung mit Eis gab man zu der Lösung von 50,0 g (0,247 Mol) 3-Amino-4-chlor-1-methyl-1H-pyrrol-2-carbonsäureethylester in 300 ml trockenem Dimethylformamid 3,7 g 80proz. Natriumhydrid, ließ die Mischung auf 10 °C kommen und tropfte unter Einhaltung dieser Temperatur die Lösung von 35,0 g (0,248 Mol) 2-Fluornitrobenzol in 200 ml wasserfreiem Dimethylformamid zu. Zu der entstandenen violettfarbigen Lösung gab man abermals 3,7 g einer 80proz. Natriumhydrid-Dispersion und rührte 30 Minuten bei Zimmertemperatur. Dann wurde die Reaktionsmischung in 3 l Eiswasser eingerührt und mit Essigsäure neutralisiert. Der Niederschlag wurde abgenutscht, gründlich mit Wasser gewaschen und zweimal aus Methanol umkristallisiert. Man erhielt 54,0 g (68 % der Theorie) an gelben Kristallen vom Fp. 130-131 °C.

e.) 3-[(2-Aminophenyl)amino]-4-chlor-1-methyl-1H-pyrrol-2-carbonsäureethylester

Die Lösung von 35,6 g (0,11 Mol) 4-Chlor-1-methyl-3-[(2-nitrophenyl)amino]-1H-pyrrol-2-carbonsäureethylester in 700 ml Tetrahydrofuran wurde nach Zusatz von 10 g Raney-Nickel 10 Stunden bei einem Wasserstoffdruck von 10 bar und einer Temperatur von 40 °C hydriert. Nach Zugabe von weiteren 10 g Raney-Nickel wurde die Hydrierung unter den gleichen Bedingungen noch 13 Stunden fortgesetzt, wonach in der Mischung Ausgangsmaterial dünnschichtchromatographisch nicht mehr nachzuweisen war. Der Katalysator wurde abfiltriert, das Filtrat im Vakuum eingeengt, der sirupöse Rückstand einmal aus tert.Butyl-methylether und einmal aus Methanol umkristallisiert. Die farblosen Kristalle schmolzen bei 104 bis 105 °C. Ausbeute 27,5 g (85 % der Theorie).

f.) 3-Chlor-1-methyl-1,4,9,10-tetrahydropyrrolo[3,2-b][1,5]-benzodiazepin-10-on

Das Gemisch aus 27,4 g (0,0933 Mol) 3-[(2-Aminophenyl)-amino]-4-chlor-1-methyl-1H-pyrrol-2-carbonsäureethylester und 90 ml 85proz. Phosphorsäure wurde im Wasserstrahlvakuum unter Rühren 2 Stunden auf 110 °C erhitzt. Die noch heiße Reaktionsmischung wurde anschließend in 1 l Eiswasser eingerührt und mit wässeriger Ammoniak-Lösung auf pH 6 gebracht. Der anfallende Feststoff schmolz nach dem Umkristallisieren aus Acetonitril bei 172-175 °C. Ausbeute : 23,0 g (99 % der Theorie).

g.) 3-Chlor-4-chloracetyl-1-methyl-1,4,9,10-tetrahydropyrrolo[3,2-b][1,5]benzodiazepin-10-on

1,7 g (6,86 mMol) 3-Chlor-1-methyl-1,4,9,10-tetrahydropyrrolo[3,2-b][1,5]benzodiazepin-10-on wurden im Gemisch mit 50 ml Acetonitril und 4,0 ml (52,9 mMol) Chloracetylchlorid 1 Stunde unter Rückfluß gekocht. Man engte im Vakuum ein, nahm den Rückstand in 20 ml Wasser auf, filtrierte und chromatographierte den festen Rückstand unter Verwendung von Dichlormethan/Cyclohexan/Essigsäureethylester (1 : 2 : 1 v/v) als Eluens. Aus den Eluaten isolierte man einen weinroten Feststoff, der nach dem Reinigen mit tert.Butyl-methylether farblose Kristalle vom Fp. 284-285 °C (Z.) lieferte. Ausbeute 1,4 g (63 % der Theorie).

h.) 3-Chlor-4-[[2-[(dimethylamino)methyl]-1-piperidinyl]-acetyl]-1-methyl-1,4,9,10-tetrahydropyrrolo[3,2-b][1,5]-benzodiazepin-10-on

Das Gemisch aus 8,0 g (0,0247 Mol) 3-Chlor-4-(chloracetyl)-1-methyl-1,4,9,10-tetrahydropyrrolo[3,2-b][1,5]benzodiazepin-10-on, 100 ml Acetonitril und 5,7 g (0,04 Mol) D,L-2-[(Dimethylamino)methyl]piperidin wurde 3 Stunden unter Rückfluß gekocht ; anschließend gab man weitere 5,0 g (0,035 Mol) D,L-2[(Dimethylamino)methyl]piperidin zu und kochte abermals 2 Stunden unter Rückfluß. Man setzte 1,0 g Aktivkohle zu, filtrierte die siedend heiße Mischung und ließ erkalten. Nach 2-stündigem Stehen des Filtrats bei Zimmertemperatur waren 4,9 g (46 % der Theorie) an farblosen Kristallen vom Fp. 226-228 °C ausgefallen. Die Mutterlauge wurde mit 100 ml Dichlormethan verdünnt, nacheinander mit 1proz. wässeriger Natronlauge, gesättigter Natriumhydrogencarbonat-Lösung und Wasser gewaschen, über Natriumsulfat getrocknet und eingedampft. Der Rückstand schmolz nach dem Umkristallisieren aus Acetonitril bei 229-230 °C. Ausbeute 3,5 g (32 % der Theorie). Nach dünnschichtchromatographischer Untersuchung (Polygram(R) SIL G/$UV_{254}$ ; Fließmittel : Dichlormethan/Cyclohexan/Methanol/konz. Ammoniak 20,4 : 4,6 : 4,6 : 0,12 ; $R_F$ 0,1 bis 0,3) enthielten beide Fraktionen nicht identische Anteile an 2 Diastereomeren. Gesamtausbeute : 78 % der Theorie.

Beispiel 2

3-Chlor-4-[[2-(diethylamino)methyl]-1-piperidinyl]acetyl]-1-methyl-1,4,9,10-tetrahydropyrrolo[3,2-b][1,5]benzodiazepin-10-on

Das Gemisch aus 7,8 g (0,024 Mol) 3-Chlor-4-(chloracetyl)-1-methyl-1,4,9,10-tetrahydropyrrolo[3,2-b][1,5]benzodiazepin-10-on, 200 ml Acetonitril, 5,1 g (0,03 Mol) D,L-2-[(Diethylamino)methyl]piperidin und 3,3 g (0,031 Mol) Natriumcarbonat wurde 5 Stunden unter Rühren und Rückfluß gekocht. Man gab 1 g Aktivkohle zu, filtrierte heiß und chromatographierte den Abdampfrückstand des Filtrats an Kieselgel unter Verwendung von Dichlormethan/Essigsäureethylester/Cyclohexan/Methanol/konz. Ammoniak

(6,08 : 2 : 0,92 : 0,92 : 0,12) zum Eluieren. Nach Eindampfen der entsprechenden Fraktionen erhielt man einen Eluatrückstand, der aus tert.Butyl-methylether und n-Propanol umkristallisiert wurde. Farblose Kristalle vom Fp. 201 °C. Ausbeute : 6,0 g (55 % der Theorie). Die dünnschichtchromatographische Untersuchung unter den in Beispiel 1 h angegebenen Bedingungen zeigte, daß die Verbindung in Form eines Gemischs zweier Diastereomerer im ungefähren Mengenverhältnis 1 : 2 vorlag. ($R_F$ 0,2 bis 0,3).

Beispiel 3

4-[[2-[(Dimethylamino)methyl]-1-piperidinyl]acetyl]-1-methyl-1,4,9,10-tetrahydropyrrolo[3,2-b][1,5]benzodiazepin-10-on

3,5 g (8,14 mMol) 3-Chlor-4-[[2-[dimethylamino)methyl]-1-piperidinyl]acetyl]-1-methyl-1,4,9,10-tetrahydropyrrolo[3,2-b][1,5]benzodiazepin-10-on wurden in 350 ml heißem Ethanol gelöst und nach Zusatz von 3 g Palladium auf Tierkohle (20proz.) 20 Stunden bei einem Wasserstoffdruck von 50 bar und einer Temperatur von 40 °C hydriert. Man filtrierte vom Katalysator ab, engte das Filtrat im Vakuum ein, nahm das kristalline Hydrochlorid in 20 ml Wasser auf, stellte die erhaltene Lösung natronalkalisch und extrahierte erschöpfend mit Dichlormethan. Die vereinigten Extrakte wurden über Natriumsulfat getrocknet und eingedampft und der verbleibende Rückstand einmal aus Ethylacetat und einmal aus Acetonitril umkristallisiert. Man erhielt 1,7 g (53 % der Theorie) an farblosen Kristallen vom Fp. 163-165 °C.

Beispiel 4

4-[[2-[(Diethylamino)methyl]-1-piperidinyl]acetyl]-1-methyl-1,4,9,10-tetrahydropyrrolo[3,2-b][1,5]benzodiazepin-10-on

Hergestellt analog Beispiel 3 aus 3-Chlor-4-[[2-[(diethylamino)methyl]-1-piperidinyl]acetyl]-1-methyl-1,4,9,10-tetrahydropyrrolo[3,2-b][1,5]benzodiazepin-10-on in einer Ausbeute von 41 % der Theorie. Fp. 141-144 °C (Acetonitril). Die dünnschichtchromatographische Untersuchung unter den in Beispiel 1 h angegebenen Bedingungen spricht für das Vorliegen der Verbindung in 2 diastereomeren Formen ($R_F$ 0,2 bis 0,3).

Beispiel 5

5,11-Dihydro-11-[[2-[(dimethylamino)methyl]-1-piperidinyl]-acetyl]-6H-pyrido[2,3-b][1,4]benzodiazepin-6-on

Hergestellt analog Beispiel 2 aus 11-(Chloracetyl)-5,11-dihydro-6H-pyrido-[2,3-b][1,4]benzodiazepin-6-on und D,L-2-[(Dimethylamino)methyl]piperidin in einer Ausbeute von 43 % der Theorie. Fp. 189-190 °C (Acetonitril unter Verwendung von Aktivkohle).

Beispiel 6

11-[3-[2-[(Diethylamino)methyl]-1-piperidinyl]-1-oxopropyl]-5,11-dihydro-6H-pyrido[2,3-b]-[1,4]benzodiazepin-6-on

4,7 g (0,0156 Mol) 11-(3-Chlor-1-oxopropyl)-5,11-dihydro-6H-pyrido[2,3-b][1,4]benzodiazepin-6-on wurden in 50 ml Acetonitril gelöst und nach Zugabe von 3,4 g (0,02 Mol) 2-[(Diethylamino)methyl]piperidin und 2,8 ml (0,03 Mol) Triethylamin 1 Stunde unter Rühren und unter Rückfluß gekocht. Man gab abermals 1,0 g (0,059 Mol) 2-[(Diethylamino)methyl]-piperidin zu und erhitzte weitere 4 Stunden auf Rückflußtemperatur. Die erkaltete Mischung wurde eingeengt, der Rückstand zwischen Wasser und Dichlormethan verteilt, die Dichlormethanphase über Natriumsulfat getrocknet, eingedampft und an Kieselgel unter Verwendung von Acetonitril/Dichlormethan/Ethylacetat/Cyclohexan/Methanol/konz. Ammoniak (6 : 3,5 : 1,5 : 0,46 : 0,46 : 0,06) zum Eluieren säulenchromatographisch gereinigt. Nach dem Umkristallisieren aus Acetonitril unter Verwendung von Aktivkohle erhielt man farblose Kristalle vom Fp. 160-162 °C. Ausbeute : 3,8 g (56 % der Theorie).

Beispiel 7

5,11-Dihydro-11-[[2-[2-(dimethylamino)ethyl]-1-piperidinyl]-acetyl]-6H-pyrido[2,3-b][1,4]benzodiazepin-6-on

Hergestellt analog Beispiel 6 aus 11-(Chloracetyl)-5,11-dihydro-6H-pyrido[2,3-b][1,4]benzodiazepin-6-on und 2-[2-(Dimethylamino)ethyl]piperidin in Gegenwart von Triethylamin in einer Ausbeute von 11 % der Theorie. Farblose Kristalle vom Fp. 181-183 °C (Acetonitril).

Entsprechend wurden erhalten :

5,11-Dihydro-11-[[2-[(ethylmethylamino)methyl]-1-piperidinyl]acetyl]-6H-pyrido[2,3-b][1,4]benzodiazepin-6-on, Fp. 200-202 °C (Acetonitril) ;

11-[[2-[(Diethylamino)methyl]-hexahydro-1H-azepin-1-yl]-acetyl]-5,11-dihydro-6H-pyrido[2,3-b][1,4]benzodiazepin-6-on ;

5,10-Dihydro-5-[[2-[(ethylmethylamino)methyl]-1-piperidinyl]acetyl]-11H-dibenzo[b,e][1,4]diazepin-11-on, Fp. 154-156 °C (Acetonitril/Essigsäureethylester 1 : 1 v/v) ;

5,11-Dihydro-11-[[2-[[(2-hydroxyethyl) (methyl)amino]methyl]-1-piperidinyl]acetyl]-6H-pyrido[2,3-b][1,4-]benzodiazepin-6-on, Fp. 176-177 °C (Acetonitril) ;

5,10-Dihydro-5-[[2-[[(2-hydroxyethyl) (methyl)amino]methyl]-1-piperidinyl]acetyl]-11H-dibenzo[b,e][1,4]diazepin-11-on, Fp. 133-134 °C (Essigsäureethylester/Aktivkohle) ;

5,11-Dihydro-11-[[2-[[(methyl) (2-methylpropyl)amino]methyl]-1-piperidinyl]acetyl]-6H-pyrido[2,3-b][1,4]benzodiazepin-6-on ;

5,10-Dihydro-5-[[2-[[(methyl) (2-methylpropyl)amino]methyl]-1-piperidinyl]acetyl]-11H-dibenzo[b,e][1,4]diazepin-11-on ;

5,11-Dihydro-11-[[2-[[methylpropylamino]methyl]-1-piperidinyl]acetyl]-6H-pyrido[2,3-b][1,4]benzodiazepin-6-on ;

5,10-Dihydro-5-[[2-[[methylpropylamino]methyl]-1-piperidinyl]acetyl]-11H-dibenzo[b,e][1,4]diazepin-11-on ;

11-[[2-[[Butylmethylamino]methyl]-1-piperidinyl]acetyl]-5,11-dihydro-6H-pyrido[2,3-b][1,4]benzodiazepin-6-on, Fp. 155-156 °C (Acetonitril) ;

5-[[2-[[Butylmethylamino]methyl]-1-piperidinyl]acetyl]-5,10-dihydro-11H-dibenzo[b,e][1,4]diazepin-11-on, Fp. 135-136 °C (Essigsäureethylester/Aktivkohle) ;

5,11-Dihydro-11-[[3-[(dimethylamino)methyl]-4-morpholinyl]-acetyl]-6H-pyrido[2,3-b][1,4]benzodiazepin-6-on ;

5,10-Dihydro-5-[[3-[(dimethylamino)methyl]-4-morpholinyl]-acetyl]-11H-dibenzo[b,e][1,4]diazepin-11-on ;

5-[[2-[(Diethylamino)methyl]-hexahydro-1H-azepin-1-yl]-acetyl]-5,10-dihydro-11H-dibenzo[b,e][1,4]diazepin-11-on ;

5,11-Dihydro-11-[[2-[(1-pyrrolidinyl)methyl]-hexahydro-1H-azepin-1-yl]acetyl-6H-pyrido[2,3-b][1,4]benzodiazepin-6-on ;

5,10-Dihydro-5-[[2-[(1-pyrrolidinyl)methyl]-hexahydro-1H-azepin-1-yl]acetyl]-11H-dibenzo[b,e][1,4]diazepin-11-on ;

5,11-Dihydro-11-[[2-[(4-methyl-1-piperazinyl)methyl]-hexahydro-1H-azepin-1-yl]acetyl]-6H-pyrido[2,3-b][1,4]benzodiazepin-6-on ;

5,10-Dihydro-5-[[2-[(4-methyl-1-piperazinyl)methyl]-hexahydro-1H-azepin-1-yl]acetyl]-11H-dibenzo[b,e][1,4]diazepin-11-on.

## Beispiel 8

11-[[2-[2-(Diethylamino)ethyl]-1-piperidinyl]acetyl]-5,11-dihydro-6H-pyrido[2,3-b][1,4]benzodiazepin-6-on

Hergestellt analog Beispiel 2 aus 11-(Chloracetyl)-5,11-dihydro-6H-pyrido[2,3-b][1,4]benzodiazepin-6-on und 2-[2-(Diethylamino)ethyl]-piperidin in einer Ausbeute von 41 % der Theorie. Farblose Kristalle vom Fp. 137-139 °C (Diisopropylether).

## Beispiel 9

5,11-Dihydro-11-[[2-[(1-pyrrolidinyl)methyl]-1-piperidinyl]-acetyl]-6H-pyrido[2,3-b][1,4]benzodiazepin-6-on

Das Gemisch aus 8,6 g (0,0299 Mol) 11-(Chloracetyl)-5,11-dihydro-6H-pyrido[2,3-b][1,4]benzodiazepin-6-on, 3,0 g (0,0283 Mol) Natriumcarbonat, 250 ml Ethanol und 5,9 g (0,035 Mol) 2-[(1-Pyrrolidinyl)methyl]piperidin wurde 9 Stunden unter Rückfluß gekocht. Dann wurde filtriert und das Filtrat im Vakuum eingedampft. Der kristallisierende Rückstand wurde einmal aus Isopropanol unter Verwendung von Tierkohle und einmal aus viel Acetonitril umkristallisiert. Farblose Kristalle vom Fp. 230-231 °C.

Ausbeute : 3,2 g (26 % der Theorie).

## Beispiel 10

5,11-Dihydro-11-[[2-[(4-morpholinyl)methyl]-1-piperidinyl]-acetyl]-6H-pyrido[2,3-b][1,4]benzodiazepin-6-on

Hergestellt analog Beispiel 2 aus 11-(Chloracetyl)-5,11-dihydro-6H-pyrido[2,3-b][1,4]benzodiazepin-6-on und 2-[(4-Morpholinyl)methyl]piperidin in einer Ausbeute von 54 % der Theorie. Farblose Kristalle vom Fp. 203-205 °C (Acetonitril unter Verwendung von Aktivkohle).

Beispiel 11

11-[[2-[[(Cyclohexyl) (methyl)amino]methyl]-1-piperidinyl]-acetyl]-5,11-dihydro-6H-pyrido[2,3-b][1,4]benzodiazepin-6-on

Hergestellt analog Beispiel 2 aus 11-(Chloracetyl)-5,11-dihydro-6H-pyrido[2,3-b][1,4]benzodiazepin-6-on und 2-[[(Cyclohexyl)(methyl)amino]methyl]piperidin in einer Ausbeute von 65 % der Theorie. Farblose Kristalle vom Fp. 175-177 °C (Acetonitril unter Verwendung von Aktivkohle).

Beispiel 12

5,11-Dihydro-11-[[2-[[(1-piperidinyl)methyl]-1-piperidinyl]-acetyl]-6H-pyrido[2,3-b][1,4]benzodiazepin-6-on

Hergestellt analog Beispiel 2 aus 11-(Chloracetyl)-5,11-dihydro-6H-pyrido[2,3-b][1,4]benzodiazepin-6-on und 2-[(1-Piperidinyl)methyl]piperidin in einer Ausbeute von 50 % der Theorie. Farblose Kristalle vom Fp. 212-214 °C (Ethanol).

Beispiel 13

5,11-Dihydro-11-[[2-[(4-methyl-1-piperazinyl)-methyl]-1-piperidinyl]acetyl]-6H-pyrido[2,3-b][1,4]benzodiazepin-6-on

Hergestellt analog Beispiel 2 aus 11-(Chloracetyl)-5,11-dihydro-6H-pyrido[2,3-b][1,4]benzodiazepin-6-on und 2-[(4-Methyl-1-piperazinyl)methyl]piperidin in einer Ausbeute von 21 % der Theorie. Farblose Kristalle vom Fp. 205-205 °C (nach Umkristallisieren aus Isopropanol und aus Acetonitril). Unter den Bedingungen (HPLC, DC), die im Beispiel 24 zu einer Trennung der Diastereomeren führten, waren Isomere nicht nachweisbar.

Beispiel 14

trans-5,11-Dihydro-11-[[2-[[(4-hydroxycyclohexyl) (methyl)-amino]methyl]-1-piperidinyl]acetyl]-6H-pyrido[2,3-b][1,4]-benzodiazepin-6-on

Hergestellt analog Beispiel 2 aus 11-(Chloracetyl)-5,11-dihydro-6H-pyrido[2,3-b][1,4]benzodiazepin-6-on und trans-2-[[(4-Hydroxycyclohexyl) (methyl)amino]methyl]piperidin in einer Ausbeute von 29 % der Theorie. Farblose Kristalle vom Fp. 183-184,5 °C (Acetonitril).

Beispiel 15

5-[[2-[(Diethylamino)methyl]-1-piperidinyl]acetyl]-5,10-dihydro-11H-dibenzo[b,e][1,4]diazepin-11-on

Das Gemisch aus 5,1 g (0,0178 Mol) 4-(Chloracetyl)-5,10-dihydro-11H-dibenzo[b,e][1,4]diazepin-11-on, 3,2 g (0,0188 Mol) 2-[(Diethylamino)methyl]piperidin und 20 ml Dimethylformamid wurde 3 Stunden auf 80 °C erhitzt. Man ließ erkalten, rührte das Gemisch in 100 ml Eiswasser ein und sättigte mit fester Soda. Dann wurde erschöpfend mit Essigsäureethylester extrahiert. Die vereinigten Essigesterauszüge wurden einmal mit wenig Wasser gewaschen, über Natriumsulfat getrocknet und im Vakuum eingedampft. Der verbleibende Rückstand wurde aus Ethanol umkristallisiert. Farblose Kristalle vom Fp. 171-172 °C. Ausbeute 0,8 g (11 % der Theorie).

Beispiel 16

5-[3-[2-[(Diethylamino)methyl]-1-piperidinyl]-1-oxopropyl]-5,10-dihydro-11H-dibenzo[b,e][1,4]diazepin-11-on

Hergestellt analog Beispiel 1 h aus 5,10-Dihydro-5-(1-oxopropen-1-yl)-11H-dibenzo[b,e][1,4]diazepin-11-on und 2-[(Diethylamino)methyl]piperidin in einer Ausbeute von 50 % der Theorie. Farblose Kristalle vom Fp. 125-127 °C (Acetonitril).

Beispiel 17

5,10-Dihydro-5-[[2-[(dimethylamino)methyl]-1-piperidinyl]-acetyl]-11H-dibenzo[b,e][1,4]diazepin-11-on

Hergestellt analog Beispiel 2 aus 5-(Chloracetyl)-5,10-dihydro-11H-dibenzo[b,e][1,4]diazepin-11-on

und 2-[(Dimethylamino)methyl]piperidin in einer Ausbeute von 54 % der Theorie. Farblose Kristalle vom Fp. 123-125 °C (Diisopropylether).

Beispiel 18

5,10-Dihydro-5-[[2-[2-(dimethylamino)ethyl]-1-piperidinyl]-acetyl]-11H-dibenzo[b,e] [1,4]diazepin-11-on

Hergestellt analog Beispiel 2 aus 5-(Chloracetyl)-5,10-dihydro-11H-dibenzo[b,e] [1,4]diazepin-11-on und 2-[2-(Dimethylamino)ethyl]piperidin in einer Ausbeute von 14 % der Theorie. Farblose Kristalle vom Fp. 155-157 °C (Acetonitril).

Beispiel 19

5-[[2-[2-(Diethylamino)ethyl]-1-piperidinyl]acetyl]-5,10-dihydro-11H-dibenzo[b,e] [1,4]diazepin-11-on

Hergestellt analog Beispiel 2 aus 5-(Chloracetyl)-5,10-dihydro-11H-dibenzo[b,e] [1,4]diazepin-11-on und 2-[2-(Diethylamino)ethyl]piperidin in einer Ausbeute von 69 % der Theorie. Farblose Kristalle vom Fp. 97-98 °C (Diisopropylether).

Beispiel 20

5,10-Dihydro-5-[[2-[(1-pyrrolidinyl)methyl]-1-piperidinyl]-acetyl]-11H-dibenzo[b,e] [1,4]diazepin-11-on

Hergestellt analog Beispiel 2 aus 5-(Chloracetyl)-5,10-dihydro-11H-dibenzo[b,e] [1,4] diazepin-11-on und 2-[(1-Pyrrolidinyl)methyl]piperidin in einer Ausbeute von 15 % der Theorie. Farblose Kristalle vom Fp. 199-201 °C (Isopropanol/Aktivkohle).

Beispiel 21

5,10-Dihydro-5-[[2-[(4-morpholinyl)methyl]-1-piperidinyl]-acetyl]-11H-dibenzo[b,e] [1,4]diazepin-11-on

Hergestellt analog Beispiel 2 aus 5-(Chloracetyl)-5,10-dihydro-11H-dibenzo[b,e] [1,4]diazepin-11-on und 2-[(4-Morpholinyl)methyl]piperidin in einer Ausbeute von 41 % der Theorie. Farblose Kristalle vom Fp. 194-195 °C (nach dem Umkristallisieren aus Diisopropylether und aus Acetonitril unter Verwendung von Aktivkohle).

Beispiel 22

5-[[2-[[(Cyclohexyl) (methyl)amino]methyl]-1-piperidinyl]-acetyl]-5,10-dihydro-11H-dibenzo[b,e] [1,4]diazepin-11-on

Hergestellt analog Beispiel 2 aus 5-(Chloracetyl)-5,10-dihydro-11H-dibenzo[b,e] [1,4]diazepin-11-on und 2-[[(Cyclohexyl) (methyl)amino]methyl]piperidin in einer Ausbeute von 35 % der Theorie. Farblose Kristalle vom Fp. 146-148 °C (Acetonitril/Aktivkohle).

Beispiel 23

5,10-Dihydro-5-[[2-[(1-piperidinyl)methyl]-1-piperidinyl]-acetyl]-11H-dibenzo[b,e] [1,4]diazepin-11-on

Hergestellt analog Beispiel 2 aus 5-(Chloracetyl)-5,10-dihydro-11H-dibenzo[b,e] [1,4]diazepin-11-on und 2-[(1-Piperidinyl)methyl]piperidin in einer Ausbeute von 71 % der Theorie. Farblose Kristalle vom Fp. 220-221 °C (Acetonitril).

Beispiel 24

5,10-Dihydro-5-[[2-[(4-methyl-1-piperazinyl)methyl]-1-piperidinyl]acetyl]-11H-dibenzo[b,e] [1,4]diazepin-11-on

Hergestellt analog Beispiel 2 aus 5-(Chloracetyl)-5,10-dihydro-11H-dibenzo[b,e] [1,4]diazepin-11-on und D,L-2-[(4-Methyl-1-piperazinyl)methyl]piperidin in einer Ausbeute von 27 % der Theorie. Farblose Kristalle vom Fp. 200-202 °C (Acetonitril/Aktivkohle). Nach dünnschichtchromatographischer Untersuchung (Polygram (R) SIL G/$UV_{254}$ ; Fließmittel : Dichlormethan/Essigsäureethylester/Cyclohexan/Methanol/konz. Ammoniak 3,5 : 1,5 : : 0,46 : 0,46 : 0,06 ; $R_F$ ca. 0,25) handelte es sich um ein Isomerengemisch im ungefähren Mengenverhältnis 1 : 1. Eine präparative Trennung gelang nach folgenden Verfahren :

a.) Durch Flüssigkeitschromatographie (HPLC): Gerät: Liquid Chromatograph Series III b, Perkin-Elmer;

Detektor: LC 75 Spectrophotometer und LC 75 Autocontrol Perkin-Elmer;
Injektor: Rheodyn-Einlaßventil 7125 mit 250 µl Probenschleife;
Schreiber: Typ 561 Perkin-Elmer;
Integrator: System I Autocontrol, Spectra Physics;
Säule: Silica A 25 × 0,25 cm (polar);
Mobile Phase: Methylenchlorid + Cyclohexan + Methanol + konz. Ammoniak (200 + 210 + 40 + 0,5 v/v);
Temperatur: 21 °C;
Lauf: Isokratisch;
Flow: 0,8 ml/min;
Detektion: 270 nm;
Empfindlichkeit: 512 $A_1$ cm;
Integrator: Recorder attenuator = 1;
Papier-Vorschub: 5 mm/min.

Es wurden jeweils ca. 100 µl einer Lösung von 15,2 mg des Diastereomeren-Gemischs in 1 ml der mobilen Phase injiziert und damit jeweils ca. 1,5 mg Substanz auf die Säule gebracht.

Ergebnis: Isomeres I, t 11,1 Minuten, 46,2 % des Gemischs
Isomeres II, t 14,7 Minuten, 53,8 % des Gemischs. Isomeres I geht bei Zimmertemperatur und in der genannten mobilen Phase mit $t_{1/2} < 9$ Tage; Isomeres II mit $t_{1/2} < 6{,}5$ Tage wieder ins ursprüngliche Gemisch über.

b.) Durch Flüssigkeitschromatographie am Jobin-Yvon Chromatospac Prep unter Verwendung von Li Chroprep Si 60, 25-40 µm (Merck-Darmstadt, Art. 9390) und einem Druck von 10 bar.

Isomeres I: Fp. 196-198 °C; dünnschichtchromatographisch einheitlich (Polygram (R) SIL G/$UV_{254}$; Fließmittel: Dichlormethan/Methanol/Cyclohexan/konz.Ammoniak 180 : 40 : 40 : 1; $R_F$ 0,4). IR, UV, MS identisch mit dem von Isomer II

$^1$H-NMR ($CDCl_3$, 400 MHz): 9,6 und 10,21 (1H, austauschbarer H), 7,97 (1H, breit, ar.H); 7,60 (1H-m, ar.H); 7,1-7,55 (6H-m; ar.H); 4,0 (ca.0,5 H-d; J=17Hz); 3,85 (ca.0,5H-d; J=17Hz); 3,55 (ca.0,5H-d; J=17Hz); 3,23 (ca.0,5H-d; J=17Hz); 0,9-2,8 (22H-m; aliph.H); die Signale bei 4,0; 3,85; 3,55; 3,23 geben Hinweise auf das Vorliegen von Rotameren.

Isomeres II: Fp. 205-206 °C, dc-einheitlich ($R_F$ 0,35). $^1$H-NMR($CDCl_3$, 400 MHz): 9,94 und 9,42 (1H, austauschbarer H); 7,99 (1H, breit, ar.H); 7,58 (1H, verbreitert, ar.H); 7,1-7,52 (6H-m; ar.H); 4,02 (1H-t; J=17Hz); 3,39 (ca.0,5H-d; J=17Hz); 3,17 (ca.0,5H-d; J=17Hz), 0,9-2,85 (22H-m; aliph.H); die Signale bei 4,02; 3,39; 3,17 geben Hinweise auf das Vorliegen zweier Rotamerer.

Die Isomeren I und II sind in kristallinem Zustand und bei Temperaturen unterhalb des Fp. völlig stabil.

## Beispiel 25

trans-5,10-Dihydro-5-[[2-[[(4-hydroxycyclohexyl) (methyl)-amino]methyl]-1-piperidinyl]acetyl]-11H-dibenzo[b,e] [1,4]-diazepin-11-on

Hergestellt analog Beispiel 2 aus 5-(Chloracetyl)-5,10-dihydro-11H-dibenzo[b,e] [1,4]diazepin-11-on und trans-2-[[(4-Hydroxycyclohexyl) (methyl)amino]methyl]piperidin in einer Ausbeute von 27 % der Theorie. Farblose Kristalle vom Fp. 161-162 °C (Acetonitril).

## Beispiel 26

4,9-Dihydro-4-[[2-[(dimethylamino)methyl]-1-piperidinyl]-acetyl]-10H-thieno[3,4-b] [1,5]benzodiazepin-10-on

Hergestellt analog Beispiel 2 aus 4-(Chloracetyl)-4,9-dihydro-10H-thieno[3,4-b] [1,5]benzodiazepin-10-on und 2-[(Dimethylamino)methyl]piperidin in einer Ausbeute von 25 % der Theorie. Farblose Kristalle vom Fp. 208-209 °C (Acetonitril/-Aktivkohle).

## Beispiel 27

4-[[2-[(Diethylamino)methyl]-1-piperidinyl]acetyl]-4,9-dihydro-10H-thieno[3,4-b] [1,5] benzodiazepin-10-on

Hergestellt analog Beispiel 2 aus 4-(Chloracetyl)-4,9-dihydro-10H-thieno[3,4-b] [1,5] benzodiazepin-10-on und 2-[(Diethylamino)methyl]piperidin in einer Ausbeute von 27 % der Theorie. Farblose Kristalle vom Fp. 163-164 °C (Acetonitril).

Beispiel 28

4,9-Dihydro-4-[[2-[(1-piperidinyl)methyl]-1-piperidinyl]-acetyl]-10H-thieno[3,4-b] [1,5] benzodiazepin-10-on

Hergestellt analog Beispiel 2 aus 4-(Chloracetyl)-4,9-dihydro-10H-thieno[3,4-b] [1,5]benzodiazepin-10-on und 2-[(Diethylamino)methyl]piperidin in einer Ausbeute von 52 % der Theorie. Farblose Kristalle vom Fp. 191-195 °C (Acetonitril).

Beispiel 29

4,9-Dihydro-4-[[2-[(1-pyrrolidinyl)methyl]-1-piperidinyl]-acetyl]-10H-thieno[3,4-b] [1,5]benzodiazepin-10-on

Hergestellt analog Beispiel 2 aus 4-(Chloracetyl)-4,9-dihydro-10H-thieno[3,4-b] [1,5]benzodiazepin-10-on und 2-[(1-Pyrrolidinyl)methyl]piperidin in einer Ausbeute von 20 % der Theorie. Farblose Kristalle vom Fp. 205-207 °C (Acetonitril/Aktivkohle).

Beispiel 30

4,9-Dihydro-4-[[2-[4-morpholinyl)methyl]-1-piperidinyl]-acetyl]-10H-thieno[3,4-b] [1,5]benzodiazepin-10-on

Hergestellt analog Beispiel 2 aus 4-(Chloracetyl)-4,9-dihydro-10H-thieno[3,4-b] [1,5]benzodiazepin-10-on und 2-[(4-Morpholinyl)methyl]piperidin in einer Ausbeute von 34 % der Theorie. Farblose Kristalle vom Fp. 193-194 °C (Acetonitril/Aktivkohle).

Beispiel 31

4-[[2-[(Diethylamino)methyl]-1-piperidinyl]acetyl]-1,3-dimethyl-1,4,9,10-tetrahydropyrrolo[3,2-b] [1,5]-benzodiazepin-10-on

Die Mischung aus 2,0 g (0,0066 Mol) 4-(Chloracetyl)-1,3-dimethyl-1,4,9,10-tetrahydropyrrolo [3,2-b] [1,5]benzodiazepin-10-on, 30 ml Dimethylformamid, 1 ml (0,0071 Mol) Triethylamin und 1,7 g (0,01 Mol) 2-[(Diethylamino)methyl]piperidin wurde unter Rühren 2 Stunden lang auf 30-40 °C erwärmt. Das Lösungsmittel wurde im Wasserstrahlvakuum abdestilliert, der Rückstand in 10 ml Wasser aufgenommen, mit Kaliumcarbonat alkalisch gestellt und mit Dichlormethan erschöpfend extrahiert. Die vereinigten Methylenchlorid-Auszüge wurden über Natriumsulfat getrocknet und eingedampft. Das so erhaltene Rohprodukt wurde an Kieselgel unter Verwendung von Dichlormethan/Methanol (9 : 1 v/v) chromatographisch gereinigt. Nach dem Umkristallisieren aus Essigsäureethylester/Methanol (95 : 5) erhielt man 1,4 g (48 % der Theorie) der gesuchten Verbindung in Form farbloser Kristalle vom Fp. 176-178 °C.

Beispiel 32

11-[[2-[(Diethylamino)methyl]-1-piperidinyl]acetyl]-5,11-dihydro-6H-pyrido[2,3-b] [1,4]benzodiazepin-6-on

5,0 g (0,0174 Mol) 11-(Chloracetyl)-5,11-dihydro-6H-pyrido-[2,3-b] [1,4]benzodiazepin-6-on wurden in 100 ml wasserfreiem Dioxan gelöst und nach Zugabe von 6,0 g (0,035 Mol) 2-[(Diethylamino)methyl]piperidin 2 Stunden unter Rückfluß gekocht.

Die Mischung wurde eingedampft, der Rückstand einmal aus Ethanol und einmal aus Methanol unter Zusatz von viel Aktivkohle umkristallisiert. Man erhielt 1,5 g (20 % der Theorie) an farblosen Kristallen vom Fp. 225-226 °C.

Beispiel 33

11-[[2-[(Diethylamino)methyl]-1-piperidinyl]acetyl]-5,11-dihydro-6H-pyrido[2,3-b] [1,4]benzodiazepin-6-on

Hergestellt analog Beispiel 2 aus 11-(Chloracetyl)-5,11-dihydro-6H-pyrido[2,3-b] [1,4] benzodiazepin-6-on und 2-[(Diethylamino)methyl]piperidin in einer Ausbeute von 97 % der Theorie. Farblose Kristalle vom Fp. 225-225,5 °C (n-Propanol).

Beispiel 34

11-[[2-[(Diethylamino)methyl]-1-piperidinyl]acetyl]-5,11-dihydro-6H-pyrido[2,3-b] [1,4]benzodiazepin-6-on

Die Mischung aus 97,6 g (0,339 Mol) 11-(Chloracetyl)-5,11-dihydro-6H-pyrido[2,3-b] [1,4]benzodiaze-

pin-6-on, 64,0 g (0,376 Mol) 2-[(Diethylamino)methyl]piperidin, 36,0 g (0,34 Mol) Natriumcarbonat und 1,7 l n-Propanol wurde 5 Stunden unter Rückfluß gekocht. Der Reaktionsansatz wurde noch heiß filtriert, dann 12 Stunden bei Raumtemperatur stehen gelassen, wonach das gewünschte Produkt sich kristallin abgeschieden hatte. Die Substanz wurde abgenutscht, dreimal mit je 100 ml n-Propanol gewaschen und im Vakuum-Trockenschrank getrocknet. Man erhielt 119,5 g (84 % der Theorie) an farblosen Kristallen vom Fp. 226-229 °C.

Nach den üblichen Methoden wurden folgende Salze hergestellt :

Methansulfonat $C_{24}H_{31}N_5O_2 \cdot CH_3SO_3H$, Fp. 220-222 °C (Ethanol) ;

Fumarat $C_{24}H_{31}N_5O_2 \cdot C_4H_4O_4$,
Fp. 192-194 °C (Isopropanol) ;

Maleinat $C_{24}H_{31}N_5O_2 \cdot C_4H_4O_4$,
Fp. 175-177 °C (Isopropanol) ;

Dihydrochlorid-dihydrat $C_{24}H_{31}N_5O_2 \cdot 2HCl \cdot 2H_2O$,
Fp. 229-230 °C (Z.) (Isopropanol/Methanol 1 : 1) ;

Dihydrobromid $C_{24}H_{31}N_5O_2 \cdot 2HBr$, Fp. 249-250 °C (Ethanol)

Nach dem in Beispiel 24 angegebenen Verfahren a) ließ sich die Base nicht in Diastereomere trennen.

## Beispiel 35

11-[[3-[(Diethylamino)methyl]-1-piperidinyl]acetyl]-5,11-dihydro-6H-pyrido[2,3-b] [1,4]benzodiazepin-6-on

Zu einer Suspension von 5,2 g (0,0181 Mol) 11-Chloracetyl-5,11-dihydro-6H-pyrido[2,3-b] [1,4]benzodiazepin-6-on in 25 ml wasserfreiem Dimethylformamid tropfte man innerhalb von 2 Stunden eine Lösung von 4,6 g (0,027 Mol) 3-[(Diethylamino)-methyl]piperidin in 10 ml trockenem Dimethylformamid, rührte anschließend noch 6 Stunden bei Zimmertemperatur und ließ über Nacht stehen. Der Ansatz wurde in 200 g Eis eingerührt, mit Kaliumcarbonat alkalisch gestellt und mit Dichlormethan erschöpfend extrahiert. Die vereinigten Dichlormethan-Auszüge wurden einmal mit 20 ml Wasser gewaschen, über Magnesiumsulfat getrocknet und im Vakuum eingedampft. Nach dem Umkristallisieren aus Essigsäureethylester erhielt man 4,5 g (59 % der Theorie) an farblosen Kristallen vom Fp. 199-200 °C.

## Beispiel 36

(S)-11-[[2-[(Diethylamino)methyl]-1-pyrrolidinyl]acetyl]-5,11-dihydro-6H-pyrido[2,3-b] [1,4]benzodiazepin-6-on

Hergestellt analog Beispiel 31 aus 11-(Chloracetyl)-5,11-dihydro-6H-pyrido[2,3-b] [1,4]benzodiazepin-6-on und (S)-(+)-2-[(Diethylamino)methyl]pyrrolidin in einer Ausbeute von 35 % der Theorie. Farblose Kristalle vom Fp. 192-193 °C (Essigsäureethylester/Methanol 99 : 1 v/v) ; $[\alpha]_D^{20} = -29{,}4°$ (Ethanol).

## Beispiel 37

5,11-Dihydro-11-[(3-Dimethylamino)-1-piperidinyl]acetyl]-6H-pyrido[2,3-b] [1,4]benzodiazepin-6-on

Hergestellt analog Beispiel 35 aus 11-(Chloracetyl)-5,11-dihydro-6H-pyrido[2,3-b] [1,4]benzodiazepin-6-on und 3-(Dimethylamino)piperidin in einer Ausbeute von 83 % der Theorie. Farblose Kristalle vom Fp. 211-212 °C (Diisopropylether).

## Beispiel 38

(S)-5,11-Dihydro-11-[[2-[(1-pyrrolidinyl)methyl]-1-pyrrolidinyl]acetyl]-6H-pyrido[2,3-b] [1,4]benzodiazepin-6-on

Hergestellt analog Beispiel 31 aus 11-(Chloracetyl)-5,11-dihydro-6H-pyrido[2,3-b] [1,4]benzodiazepin-6-on und (S)-(+)-[(1-Pyrrolidinyl)methyl]pyrrolidin in einer Ausbeute von 32 % der Theorie. Farblose Kristalle vom Fp. 192-193 °C (Essigsäureethylester). $[\alpha]_D^{20} = -18{,}1°$ (Ethanol).

## Beispiel 39

5,10-Dihydro-5-[[3-(dimethylamino )-1-piperidinyl]acetyl]-11H-dibenzo[b,e] [1,4]diazepin-11-on

Hergestellt analog Beispiel 35 aus 5-(Chloracetyl)-5,10-dihydro-11H-dibenzo[b,e] [1,4]diazepin-11-on und 3-(Dimethylamino)piperidin in einer Ausbeute von 92 % der Theorie. Farblose Kristalle vom Fp. 199-200 °C (Diisopropylether).

Beispiel 40

(S)-5,10-Dihydro-5-[[2-[(1-pyrrolidinyl)methyl]-1-pyrrolidinyl] acetyl]-11H-dibenzo[b,e] [1,4]diazepin-11-on

Hergestellt analog Beispiel 31 aus 5-(Chloracetyl)-5,10-dihydro-11H-dibenzo[b,e] [1,4]diazepin-11-on und (S)-(+)-2-[(1-Pyrrolidinyl)methyl]pyrrolidin in einer Ausbeute von 18 % der Theorie. Farblose Kristalle vom Fp. 142-144 °C (Essigsäureethylester/Methanol 99 : 1). $[\alpha]_D^{20}$ = — 18,5° (Ethanol).

Beispiel 41

5-[[3-[(Diethylamino)methyl]-1-piperidinyl]acetyl-5,10-dihydro-11H-dibenzo[b,e] [1,4]diazepin-11-on-dihydrochloriddihydrat

Hergestellt analog Beispiel 35 aus 5-(Chloracetyl)-5,10-dihydro-11H-dibenzo[b,e] [1,4]diazepin-11-on und 3-[(Diethylamino)methyl]piperidin in einer Ausbeute von 39 % der Theorie. Farblose Kristalle vom Fp. 140 °C (Isopropanol/Essigsäureethylester).

Beispiel 42

(S)-5-[[2-[(Diethylamino)methyl]-1-pyrrolidinyl]acetyl]-5,10-dihydro-11H-dibenzo[b,e] [1,4]diazepin-11-on

Hergestellt analog Beispiel 31 aus 5-(Chloracetyl)-5,10-dihydro-11H-dibenzo[b,e] [1,4]diazepin-11-on und (S)-(+)-2-[(Diethylamino)methyl]pyrrolidin in einer Ausbeute von 31 % der Theorie. Farbloses, hochviskoses Öl vom $[\alpha]_D^{20}$ = — 10,5° (Ethanol).

$C_{24}H_{30}N_4O_2$ (406,53)

| | | | |
|---|---|---|---|
| Ber. : | C 70,91 | H 7,44 | N 13,78 |
| Gef. : | C 70,55 | H 7,32 | N 13,48 |

IR($CH_2Cl_2$) : NH 3370/cm ; CO 1640-1700/cm (breit)
UV (Ethanol) : $_{max}$(neutral) : 270 nm (E = 0.075) (Schulter)
$_{max}$(basisch) : 252 nm (E = 0,11) (Schulter)
290 nm (E = 0,83) (Schulter)

(c = 50 mg/l ; Schichtdicke : 2 mm) $^1$H-NMR ($CDCl_3$/$CD_3OD$ ; 400 MHz) : 7,92 (1H-d ; ar.H) ; 7,58-7,66 (1H-m ; ar.H) ; 7,1-7,54 (6H-m ; ar.H) ; 4,05-4,21(t) ; 3,8-3,9(d) ; 3,7-3,8(d) ; 3,24-3,33(d) ; 3,2-1,3(m) ; (zusammen 15 aliph.H) ; 0,85-1,05 (6H-m ; aliph.H).

Beispiel 43

(S)-5,10-Dihydro-5-[[2-[(4-methyl-1-piperazinyl)methyl]-1-pyrrolidinyl]acetyl]-11H-dibenzo[b,e] [1,4]diazepin-11-on

Hergestellt analog Beispiel 31 aus 5-(Chloracetyl)-5,10-dihydro-11H-dibenzo[b,e] [1,4]diazepin-11-on und (S)-(+)-2-[(4-Methyl-1-piperazinyl)methyl]pyrrolidin in einer Ausbeute von 27 % der Theorie. Farblose Kristalle vom Fp. 168-170 °C (Essigsäureethylester). $[\alpha]_D^{20}$ = — 18,9° (Ethanol). $R_F$ 0,33 (Merck-DC-Fertigplatten Kieselgel 60 F-254 ; Fließmittel : Dichlormethan/Methanol/Cyclohexan/konz.Ammoniak [120 : 24 : 30 : 2]). In den Mutterlaugen kann dünnschichtchromatographisch ein zweites Isomer ($R_F$ 0,30) nachgewiesen werden.

Beispiel 44

(S)-5,11-Dihydro-11-[[2-[(4-methyl-1-piperazinyl]methyl]-1-pyrrolidinyl]acetyl]-6H-pyrido[2,3-b] [1,4]benzodiazepin-6-on

Hergestellt analog Beispiel 31 aus 11-(Chloracetyl)-5,11-dihydro-6H-pyrido[2,3-b] [1,4-benzodiazepin-6-on und (S)-(+)-2-[(4-Methyl-1-piperazinyl)methyl]pyrrolidin in einer Ausbeute von 13 % der Theorie. Farblose Kristalle vom Fp. 182-185 °C (Z.) (aus Essigsäureethylester/Methanol 99 : 1 v/v). $[\alpha]_D^{20}$ = — 11,2° (Ethanol).

Beispiel 45

5,11-Dihydro-11-[[4-(dimethylamino)-1-piperidinyl]acetyl]-6H-pyrido[2,3-b] [1,4]benzodiazepin-6-on-hydrochlorid

7,0 g (0,0243 Mol) 11-(Chloracetyl)-5,11-dihydro-6H-pyrido-[2,3-b] [1,4]benzodiazepin-6-on wurden in 150 ml wasserfreiem Dioxan suspendiert und nach Zugabe von 4,9 g (0,0298 Mol) 4-Dimethylamino)-piperidin-hydrochlorid und 10,0 g (0,099 Mol) Triethylamin 3 Stunden unter Rückfluß gekocht. Dann wurde das Gemisch im Vakuum eingedampft, der Rückstand natronalkalisch gestellt und mit Dichlormethan erschöpfend extrahiert. Die vereinigten Dichlormethan-Auszüge wurden über Natriumsulfat getrocknet und eingedampft, der hochviskose Rückstand in Dioxan aufgenommen, mit Aktivkohle geklärt und mit etherischer Chlorwasserstofflösung versetzt. Die anfallenden farblosen Kristalle schmolzen nach dem Umkristallisieren aus Methanol bei 258 °C. Ausbeute : 2,0 g (20 % der Theorie).

Beispiel 46

11-[[2-[(Diethylamino)methyl]-1-piperidinyl]acetyl]-5,11-dihydro-6H-pyrido[2,3-b] [1,4]benzodiazepin-6-on

Man erwärmte ein Gemisch von 14,43 g (0,0632 Mol) 2-[(Diethylamino)methyl]-1-piperidinessigsäure und 2,0 g einer 75proz. Natriumhydrid-Dispersion in Paraffinöl in 160 ml Dimethylformamid bei 50 bis 80 °C so lange, bis die Wasserstoffentwicklung beendet war. Zu dem entstandenen Natriumsalz der genannten Säure fügte man 13,2 g (0,0625 Mol) 5,11-Dihydro-6H-pyrido[2,3-b] [1,4]benzodiazepin-6-on und tropfte bei —10°C 9,9 g (0,0646 Mol) Phosphoroxidchlorid innerhalb von 10 Minuten zu. Man rührte 4 Stunden bei —10 °C, 4 Stunden bei 0 °C und 20 Stunden bei Zimmertemperatur. Man rührte die Mischung in 300 g Eis ein, stellte mit Natronlauge auf pH 9 und schüttelte erschöpfend mit Dichlormethan aus. Die vereinigten organischen Phasen wurden einmal mit wenig Eiswasser gewaschen, über Natriumsulfat getrocknet und eingedampft. Der Rückstand wurde aus n-Propanol unter Verwendung von Aktivkohle umkristallisiert. Farblose Kristalle vom Fp. 226-229 °C, nach Dünnschichtchromatogramm, Mischschmelzpunkt, IR-, UV- und $^1$H-NMR-Spektrum völlig identisch mit einer nach Beispiel 34 erhaltenen Probe. Ausbeute : 4,1 g (16 % der Theorie).

Beispiel 47

5,11-Dihydro-11-[[2-[(dimethylamino)methyl]-1-piperidinyl]-acetyl]-6H-pyrido[2,3-b] [1,4]benzodiazepin-6-on

Hergestellt analog Beispiel 46 aus 2-[(Dimethylamino)-methyl]-1-piperidinessigsäure und 5,11-Dihydro-6H-pyrido-[2,3-b] [1,4]benzodiazepin-6-on in einer Ausbeute von 12 % der Theorie. Farblose Kristalle vom Fp. 189-190 °C (n-Propanol), nach Dünnschichtchromatogramm, Mischschmelzpunkt und IR-Spektrum identisch mit einer nach Beispiel 5 erhaltenen Probe.

Beispiel 48

11-[[2-[(Diethylamino)methyl]-1-piperidinyl]acetyl]-5,11-dihydro-6H-pyrido[2,3-b] [1,4]benzodiazepin-6-on

Zu einer Suspension von 22,83 g (0,1 Mol) 2-[(Diethylamino)-methyl]-1-piperidinessigsäure in 200 ml trockenem Tetrahydrofuran wurden bei 0 °C 11,0 g (0,101 Mol) Chlorkohlensäureethylester zugetropft. Man fügte 21,12 g (0,1 Mol) 5,11-Dihydro-6H-pyrido[2,3-b] [1,4]benzodiazepin-6-on und 20,24 g (0,2 Mol) Triethylamin zu der entstandenen Suspension, rührte noch eine Stunde bei 0 °C und anschließend 4 Stunden bei Zimmertemperatur. Man goß in 1,6 l 2 N Natronlauge ein, extrahierte erschöpfend mit Dichlormethan, engte die organische Phase zur Trockne ein und reinigte den Rückstand durch Umkristallisieren aus Ethanol und aus Methanol. Man erhielt 6,3 g (15 % der Theorie) an farblosen Kristallen vom Fp. 226-228 °C, nach Dünnschichtchromatogramm, Mischschmelzpunkt und IR-Spektrum völlig identisch mit einer nach Beispiel 34 hergestellten Probe.

Beispiel 49

4-[[2-[(Dimethylamino)methyl]-1-piperidinyl]acetyl]-1-methyl-1,4,9,10-tetrahydropyrrolo[3,2-b] [1,5]benzodiazepin-10-on

4,17 g (9,7 mMol) 3-Chlor-4-[[2-[(dimethylamino)methyl]-1-piperidinyl]acetyl]-1-methyl-1,4,9,10-tetrahydropyrrolo[3,2-b] [1,5]benzodiazepin-10-on wurden in einem Gemisch aus 5 ml 85proz. Ameisensäure und 25 ml Dimethylformamid gelöst und nach Zusatz von 0,5 g 10proz. Palladium/Aktivkohle 3 Stunden unter Rückfluß gekocht. Man gab 7,0 ml Ameisensäure zu, kochte weiter 6 Stunden unter Rückfluß und erhitzte nach Versetzen mit weiteren 4,0 ml Ameisensäure und 0,8 g 10proz. Palladium/Aktivkohle abschließend nochmals 8 Stunden unter Rückfluß. Die Mischung wurde heiß filtriert, das Filtrat im Vakuum eingedampft und der Rückstand säulenchromatographisch (Kieselgel ; Dichlormethan/Essigsäureethylester/Methanol/konz. Ammoniak 3,5 : 1,5 : 0,46 : 0,06 v/v) gereinigt. Man erhielt 1,3 g (34 % der Theorie) an farblosen Kristallen vom Fp. 163-165 °C (Acetonitril), nach Dünnschichtchromatogramm und IR-, UV- und $^1$H-NMR-Spektren identisch mit einem nach Beispiel 3 erhaltenen Präparat.

Beispiel 50

4-[[2-[(Diethylamino)methyl]-1-piperidinyl]acetyl]-1-methyl-1,4,9,10-tetrahydropyrrolo[3,2-b] [1,5]benzodiazepin-10-on

Die Mischung aus 4,58 g (0,01 Mol) 3-Chlor-4-[[2-[(diethylamino)methyl]-1-piperidinyl]acetyl]-1-methyl-1,4,9,10-tetrahydropyrrolo[3,2-b] [1,5]benzodiazepin-10-on, 83,3 mg (0,001 Mol) 2 : 1-Tris(o-tolyl)-phosphin-Palladiumacetat-Katalysator, 2,025 g (0,044 Mol) Ameisensäure und 5,77 g (0,057 Mol) Triethylamin in 200 ml Tetrahydrofuran wurde unter Stickstoff-Atmosphäre im Autoklaven 40 Stunden auf 100 °C erhitzt. Die Mischung wurde filtriert und im Vakuum eingedampft, der Rückstand natronalkalisch gestellt und erschöpfend mit Dichlormethan extrahiert. Die getrockneten und eingedampften organischen Phasen wurden wie in Beispiel 49 säulenchromatographisch gereinigt. Man erhielt 1,55 g (37 % der Theorie) an farblosen Kristallen vom Fp. 141-144 °C (Acetonitril), nach Dünnschichtchromatogramm und IR-Spektruum identisch mit einer nach Beispiel 4 erhaltenen Probe.

Beispiel 51

(S)-5,11-Dihydro-11-[[2[(4-morpholinyl)methyl]-1-pyrrolidinyl]acetyl]-6H-pyrido[2,3-b] [1,4]benzodiazepin-6-on

Hergestellt analog Beispiel 31 aus 11-(Chloracetyl)-5,11-dihydro-6H-pyrido[2,3-b] [1,4]benzodiazepin-6-on und (S)-4-[(2-Pyrrolidinyl)methyl]morpholin in einer Ausbeute von 10 % der Theorie. Farblose Kristalle vom Fp. 202-203 °C (Essigsäureethylester/Methanol 1 : 1 v/v). $[\alpha]_D^{20}$ — 2,93° (Ethanol).

Beispiel 52

(+)-11-[[2-[(Diethylamino)methyl]-1-piperidinyl]acetyl]-5,11-dihydro-6H-pyrido[2,3-b] [1,4]benzodiazepin-6-on

a.) (—)-2-[(Diethylamino)methyl]piperidin

Man vereinigte die Lösungen von 45,2 g (0,265 Mol) racemischem 2-[(Diethylamino)methyl]piperidin in 132 ml Methanol und von 93,0 g (0,62 Mol) L-(+)-Weinsäure in 264 ml Methanol, ließ das Gemisch über Nacht bei Zimmertemperatur stehen nutschte den entstandenen Niederschlag ab, wusch ihn mit Methanol durch und kochte ihn mit 250 ml Methanol 30 Minuten lang aus. Man kristallisierte insgesamt viermal aus Ethanol/Wasser (4 : 1 v/v) um und erhielt 29,9 g (48 % der Theorie) an farblosen Kristallen vom Fp. 191,0-192,5 °C, die als das Ditartrat identifiziert werden konnten.

$C_{10}H_{22}N_2 \times 2C_4H_6O_6$ (470,48)

| | | | |
|---|---|---|---|
| Ber. : | C 45,95 | H 7,28 | N 5,95 |
| Gef. : | C 46,06 | H 7,08 | N 5,96 |

Man setzte mit Kalilauge um und erhielt nach üblicher Aufarbeitung die gesuchte freie Base vom Siedepunkt 17 mm Hg 88-94 °C ; $[\alpha]_D^{20}$ — 68° (Ethanol). Zur Gehaltsbestimmung wurde eine Probe der Base mit (S)-(—)-1-Phenylethylisocyanat in den entsprechenden Harnstoff übergeführt und anschließend mittels HPLC untersucht. Der Gehalt der Base an (—)-Enantiomerem lag danach bei mindestens 98,9 %.

b.) (+)-11-[[2-[(Diethylamino)methyl]-1-piperidinyl]acetyl]-5,11-dihydro-6H-pyrido[2,3-b] [1,4]benzodiazepin-6-on

Hergestellt analog Beispiel 2 aus 11-(Chloracetyl-5,11-dihydro-6H-pyrido[2,3-b] [1,4]benzodiazepin-6-on und (—)-2-[(Diethylamino)methyl]piperidin in einer Ausbeute von 59 % der Theorie. Farblose Kristalle vom Fp. 210-211,5 °C (n-Propanol) ; $[\alpha]_D^{20}$ + 11,4° (verdünnte wässerige Salzsäure). Das Dihydrobromid schmilzt bei 241-242 °C (unter Zersetzung ; aus Ethanol).

Beispiel 53

(—)-11-[[2-[(Diethylamino)methyl]-1-piperidinyl]acetyl]-5,11-dihydro-6H-pyrido[2,3-b] [1,4]benzodiazepin-6-on

a.) (+)-2-[(Diethylamino)methyl]piperidin
Hergestellt analog Beispiel 52a) aus racemischem 2-[(Diethylamino)methyl]piperidin und D-(—)-Weinsäure. Das Ditartrat schmolz bei 191-192,5 °C. Der durch Umsetzung mit (S)-(—)-1-Phenylethylisocyanat und anschließende HPLC-Untersuchung ermittelte Gehalt der Base vom Siedepunkt 2,27 kPa (17 mmHg) 88-94 °C an (+)-Enantiomeren lag bei 98,5 % ; $[\alpha]_D^{20}$ — 64° (Ethanol).

b. (—)-11-[[2-[(Diethylamino)methyl]-1-piperidinyl]acetyl]-5,11-dihydro-6H-pyrido[2,3-b] [1,4] benzodiazepin-6-on

Hergestellt analog Beispiel 2 aus 11-(Chloracetyl)-5,11-dihydro-6H-pyrido [2,3-b] [1,4] benzodiazepin-6-on und (+)-2-[(Diethylamino)methyl]piperidin in einer Ausbeute von 71 % der Theorie. Farblose Kristalle vom Fp. 210-211,5 °C (n-Propanol/Aktivkohle). $[\alpha]_D^{20}$ — 12° (verdünnte wässerige Salzsäure).

Beispiel 54

11-[[3-(Diethylamino)-hexahydro-1H-azepin-1-yl]acetyl]-5,11-dihydro-6H-pyrido[2,3-b] [1,4]benzodiazepin-6-on

Hergestellt analog Beispiel 2 aus 11-(Chloracetyl)-5,11-dihydro-6H-pyrido[2,3-b][1,4]benzodiazepin-6-on und 3-(Diethylamino)-hexahydro-1H-azepin in einer Ausbeute von 53 % der Theorie. Farblose Kristalle vom Fp. 222-224 °C (n-Propanol).

Beispiel 55

4-[[2-[(Diethylamino)methyl]-1-piperidinyl]acetyl]-4,9-dihydro-3-methyl-10H-thieno[3,4-b][1,5]benzodiazepin-10-on

Hergestellt analog Beispiel 2 aus 4-(Chloracetyl)-4,9-dihydro-3-methyl-10H-thieno[3,4-b][1,5]benzodiazepin-10-on und 2-[(Diethylamino)methyl]piperidin in einer Ausbeute von 26 % der Theorie. Farblose Kristalle vom Fp. 147-149 °C (Cyclohexan/Essigsäureethylester 9 : 1 v/v).

Beispiel 56

4,9-Dihydro-4-[[2-[(dimethylamino)methyl]-1-piperidinyl]-acetyl]-3-methyl-10H-thieno[3,4-b][1,5]benzodiazepin-10-on

Hergestellt analog Beispiel 2 aus 4-(Chloracetyl)-4,9-dihydro-3-methyl-10H-thieno[3,4-b][1,5]benzodiazepin-10-on und 2-[(Dimethylamino)methyl]piperidin in einer Ausbeute von 22 % der Theorie. Farblose Kristalle vom Fp. 172-173,5 °C (Cyclohexan/Essigsäureethylester 4 : 1 v/v).

Beispiel 57

11-[[2-[(Diethylamino)methyl]-1-piperidinyl]acetyl]-5,11-dihydro-6H-pyrido[2,3-b][1,4]benzodiazepin-6-on

Die Suspension aus 276 g (0,959 Mol) 11-(Chloracetyl)-5,11-dihydro-6H-pyrido[2,3-b][1,4]benzodiazepin-6-on, 244,95 g (1,007 Mol) 2-[(Diethylamino)methyl]piperidin-dihydrochlorid, 223,9 g (2,112 Mol) Natriumcarbonat und 1,92 l Acetonitril wurde 6 Stunden bei einer Reaktionstemperatur von 70 °C gerührt. Nach dieser Zeit war das 11-(Chloracetyl)-5,11-dihydro-6H-pyrido[2,3-b][1,4]benzodiazepin-6-on zu 95-97 % umgesetzt (DC). Man ließ über Nacht bei Zimmertemperatur stehen, nutschte ab und wusch den Nutscheninhalt dreimal mit je 60 ml Acetonitril gründlich durch. Die dunkel gefärbten Filtrate wurden verworfen ; der zurückgebliebene Feststoff wurde 2 Stunden lang mit 1,5 l Wasser verrührt, wiederum abgenutscht und mit weiteren 300 ml Wasser gewaschen, schließlich im Umlufttrockenschrank bei 40 °C getrocknet. Das so erhaltene rohe Produkt (390 g) wurde in einen 10-l-Kolben übergeführt, in 5,14 l 1-Propanol aufgenommen und nach Zugabe von 13,5 g (0,0555 Mol) 2-[(Diethylamino)methyl]piperidin-dihydrochlorid und 11,8 g (0,1113 Mol) wasserfreiem Natriumcarbonat 16 Stunden unter Rückfluß gekocht. Nach Zugabe von 39 g Aktivkohle wurde abermals 30 Minuten zum Sieden erhitzt und die siedend heiße Mischung anschließend durch SEITZ-Filter gedrückt. Die Klärung wurde in ähnlicher Weise unter Verwendung von jeweils 20 g Aktivkohle noch zweimal wiederholt. Das Filtrat ließ man 12 Stunden bei Raumtemperatur stehen, nutschte die ausgefallenen Kristalle ab und wusch sie zweimal mit je 50 ml 1-Propanol. Das Produkt wurde abermals mit 0,5 l kaltem 1-Propanol verrührt, abgesaugt und erneut mit zweimal je 50 ml 1-Propanol gewaschen. Man trocknete im Umlufttrockenschrank bei 40 °C und erhielt 263 g (65 % der Theorie) an fast farblosen Kristallen vom Fp. 226-227 °C.

Beispiel 58

5,10-Dihydro-5-[[3-(1-methyl-2-pyrrolidinyl)-1-piperidinyl]-acetyl]-11H-dibenzo[b,e][1,4]diazepin-11-on

Hergestellt analog Beispiel 35 aus 5-(Chloracetyl)-5,10-dihydro-11H-dibenzo[b,e][1,4]diazepin-11-on und Hexahydronicotin in einer Ausbeute von 24 % der Theorie. Farblose Kristalle vom Fp. 161-163 °C (Essigsäureethylester).

Beispiel 59

5,11-Dihydro-11-[[3-(1-methyl-2-pyrrolidinyl)-1-piperidinyl]-acetyl]-6H-pyrido[2,3-b][1,4]benzodiazepin-6-on

Hergestellt analog Beispiel 35 aus 11-(Chloracetyl)-5,11-dihydro-6H-pyrido[2,3-b][1,4]benzodiazepin-6-on und Hexahydronicotin in einer Ausbeute von 11,5 % der Theorie. Farblose Kristalle vom Fp. 203 °C (Diisopropylether).

Beispiel 60

5-[[3-[[(Cyclohexyl) (methyl)amino]methyl]-1-piperidinyl]-acetyl]-5,10-dihydro-11H-dibenzo[b,e][1,4]diazepin-11-on

Hergestellt analog Beispiel 35 aus 5-(Chloracetyl)-5,10-dihydro-11H-dibenzo[b,e][1,4]diazepin-11-on und 3-[[(Cyclohexyl) (methyl)amino]methyl]piperidin in einer Ausbeute von 62 % der Theorie. Farblose Kristalle vom Fp. 195-196 °C (Essigsäureethylester/1,2-Dichlorethan/Diisopropylether 1 : 1 : 1 v/v/v).

Beispiel 61

11-[[3-[[(Cyclohexyl) (methyl)amino]methyl]-1-piperidinyl]-acetyl]-5,11-dihydro-6H-pyrido[2,3-b][1,4]benzodiazepin-6-on

Hergestellt analog Beispiel 35 aus 11-(Chloracetyl)-5,11-dihydro-6H-pyrido[2,3-b][1,4]benzodiazepin-6-on und 3-[[(Cyclohexyl) (methyl)amino]methyl]piperidin in einer Ausbeute von 65 % der Theorie. Farblose Kristalle vom Fp. 199-200 °C (Diisopropylether).

Beispiel 62

5,10-Dihydro-5-[[3-[(dimethylamino)methyl]-1-piperidinyl]-acetyl]-11H-dibenzo[b,e][1,4]diazepin-11-on

Hergestellt analog Beispiel 35 aus 5-(Chloracetyl)-5,10-dihydro-11H-dibenzo[b,e][1,4]diazepin-11-on und 3-[[Dimethylamino]methyl]piperidin in einer Ausbeute von 81 % der Theorie. Farblose Kristalle vom Fp. 148-150 °C (Diisopropylether).

Beispiel 63

5,11-Dihydro-11-[[3-[(dimethylamino)methyl]-1-piperidinyl]-acetyl]-6H-pyrido[2,3-b][1,4]benzodiazepin-6-on

Hergestellt analog Beispiel 35 aus 11-(Chloracetyl)-5,11-dihydro-6H-pyrido[2,3-b][1,4]benzodiazepin-6-on und 3-[(Dimethylamino)methyl]piperidin in einer Ausbeute von 75 % der Theorie. Farblose Kristalle vom Fp. 189-190 °C (Diisopropylether).

Beispiel 64

5,10-Dihydro-5-[[3-[(1-pyrrolidinyl)methyl]-1-piperidinyl]-acetyl]-11H-dibenzo[b,e][1,4]diazepin-11-on

Hergestellt analog Beispiel 35 aus 5-(Chloracetyl)-5,10-dihydro-11H-dibenzo[b,e][1,4]diazepin-11-on und 3-[(1-Pyrrolidinyl)methyl]piperidin in einer Ausbeute von 69 % der Theorie. Farblose Kristalle vom Fp. 145-146 °C (Essigsäureethylester/Diisopropylether 1 : 1 v/v).

Beispiel 65

5,11-Dihydro-11-[[3-[(1-pyrrolidinyl]methyl]-1-piperidinyl]-acetyl]-6H-pyrido[2,3-b][1,4]benzodiazepin-6-on

Hergestellt analog Beispiel 35 aus 11-(Chloracetyl)-5,11-dihydro-6H-pyrido[2,3-b][1,4]benzodiazepin-6-on und 3-[(1-Pyrrolidinyl)methyl]piperidin in einer Ausbeute von 98 % der Theorie. Farblose Kristalle vom Fp. 174-176 °C (Diisopropylether).

Beispiel 66

5,11-Dihydro-11-[(1-piperidinyl)methyl]-1-piperidinyl]-acetyl]-6H-pyrido [2,3-b] [1,4]benzodiazepin-6-on

Hergestellt analog Beispiel 35 aus 11-(Chloracetyl)-5,11-dihydro-6H-pyrido[2,3-b] [1,4]benzodiazepin-6-on und 3-[(1-Piperidinyl)methyl]piperidin in einer Ausbeute von 81 % der Theorie. Farblose Kristalle vom Fp. 188-190 °C (Essigsäure-ethylester/Diisopropylether 1 : 1 v/v).

Beispiel 67

5,10-Dihydro-5-[[3-[(1-piperidinyl)methyl]-1-piperidinyl]-acetyl]-11H-dibenzo[b,e] [1,4]diazepin-11-on

Hergestellt analog Beispiel 35 aus 5-(Chloracetyl)-5,10-dihydro-11H-dibenzo[b,e][1,4]diazepin-11-on und 3-[(1-Piperidinyl)methyl]piperidin in einer Ausbeute von 69 % der Theorie. Farblose Kristalle vom Fp. 150-152 °C (Essigsäureethylester).

Beispiel 68

11-[[2-[(Diethylamino)methyl]-hexahydro-1H-azepin-1-yl]-acetyl]-5,11-dihydro-6H-pyrido[2,3-b][1,4]benzodiazepin-6-on

Hergestellt analog Beispiel 35 aus 11-(Chloracetyl)-5,11-dihydro-6H-pyrido[2,3-b] [1,4]benzodiazepin-6-on und 2-[(Diethylamino)methyl]-hexahydro-1H-azepin in einer Ausbeute von 4 % der Theorie. Farblose Kristalle vom Fp. 151-153 °C (Diisopropylether/Essigsäureethylester 98 : 2 v/v).

Beispiel 69

5-[[2-[(Diethylamino)methyl]-hexahydro-1H-azepin-1-yl]-acetyl]-5,10-dihydro-11H-dibenzo[b,e] [1,4]diazepin-11-on

Hergestellt analog Beispiel 35 aus 5-(Chloracetyl)-5,10-dihydro-11H-dibenzo[b,e] [1,4]diazepin-11-on und 2-[(Diethylamino)methyl]-hexahydro-1H-azepin in einer Ausbeute von 6 % der Theorie. Farblose Kristalle vom Fp. 118-120 °C (Diisopropylether).

Beispiel 70

5,11-Dihydro-11-[[2-[(dimethylamino-N-oxid)methyl]-1-piperidinyl]acetyl]-6H-pyrido[2,3-b] [1,4]benzodiazepin-6-on

Die Lösung von 1,5 g (3,81 mMol) 5,11-Dihydro-11-[[2-[(dimethylamino)methyl]-1-piperidinyl]acetyl]-6H-pyrido [2,3-b]-[1,4]benzodiazepin-6-on in 10 ml Methanol wurde tropfenweise mit 1 ml 35 %igem Perhydrol versetzt und 6 Stunden bei einer Temperatur von 30 °C gerührt. Das überschüssige Wasserstoffperoxid wurde durch Zugabe einer Spur Platinmohr zersetzt, die Lösung nach dem Filtrieren eingedampft, der Rückstand an 100 g Kieselgel unter Verwendung von Methylenchlorid/Methanol/Cyclohexan/konzentriertem Ammoniak im Volumenverhältnis 68 : 15 : 15 : 2 zum Eluieren chromatographisch gereinigt. Nach Eindampfen der entsprechenden Eluate und Umkristallisieren aus Essigsäureethylester und dann Acetonitril erhielt man 0,6 g (38 % der Theorie) an farblosen Kristallen vom Fp. 171-172 °C.

Beispiel 71

11-[[2-[(Diethylamin-N-oxid)methyl]-1-piperidinyl]acetyl]-5,11-dihydro-6H-pyrido [2,3-b] [1,4]benzodiazepin-6-on

Hergestellt analog Beispiel 70 aus 11-[[2-[(Diethylamino)methyl]-1-piperidinyl]-5,11-dihydro-6H-pyrido [2,3-b] [1,4]benzodiazepin-6-on und Wasserstoffperoxid in einer Ausbeute von 30 % der Theorie. Farblose Kristalle vom Fp. 186-187 °C (Acetonitril).

Im folgenden wird die Herstellung pharmazeutischer Anwendungsformen anhand einiger Beispiele beschrieben :

Beispiel I

Tabletten mit 5 mg 11-[[2-[(Diethylamino)methyl]-1-piperidinyl]acetyl]-5,11-dihydro-6H-pyrido [2,3-b] [1,4]benzodiazepin-6-on

Zusammensetzung :

1 Tablette enthält :

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren

Aus Kartoffelstärke wird durch Erwärmen ein 10 %iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45 °C getrocknet, nochmals durch obiges Sieb gerieben, mit Magnesiumstearat vermischt und zu Tabletten verpreßt.

| | |
|---|---|
| Tablettengewicht | 220 mg |
| Stempel | 9 mm |

Beispiel II

Dragées mit 5 mg 11-[[2-[(Diethylamino)methyl]-1-piperidinyl]acetyl]-5,11-dihydro-6H-pyrido [2,3-b] [1,4] benzodiazepin-6-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert. Dragéegewicht : 300 mg

Beispiel III

Ampullen mit 1 mg 11-[[2-[(Diethylamino)methyl]-1-piperidinyl]acetyl]-5,11-dihydro-6H-pyrido [2,3-b] [1,4]benzodiazepin-6-on

Zusammensetzung :

1 Ampulle enthält :

| | |
|---|---|
| Wirkstoff | 1,0 mg |
| Natriumchlorid | 8,0 mg |
| Dest. Wasser | ad 1 ml |

Herstellungsverfahren

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.
Sterilisation : 20 Minuten bei 120 °C.

Beispiel IV

Suppositorien mit 5 mg 11-[[2-[(Diethylamino)methyl]-1-piperidinyl]acetyl]-5,11-dihydro-6H-pyrido [2,3-b] [1,4]-benzodiazepin-6-on

Zusammensetzung :

1 Zäpfchen enthält :

| | |
|---|---|
| Wirkstoff | 5,0 mg |
| Zäpfchenmasse (z. B. Witepsol W 45(R)) | 1 695,0 mg |
| | 1 700,0 mg |

Herstellungsverfahren

Die feinpulverisierte Wirksubstanz wird in der geschmolzenen und auf 40 °C abgekühlten Zäpfchenmasse suspendiert. Man gießt die Masse bei 37 °C in leicht vorgekühlte Zäpfchenformen aus. Zäpfchengewicht 1,7 g.

Beispiel V

Tropfen mit 4,9-Dihydro-11-[[2-[(diethylamino)methyl]-1-piperidinyl]acetyl]-5,11-dihydro-6H-pyrido [2,3-b] [1,4]-benzodiazepin-6-on

Zusammensetzung :

100 ml Tropflösung enthalten :

| | | |
|---|---|---|
| p-Hydroxybenzoesäuremethylester | | 0,035 g |
| p-Hydroxybenzoesäurepropylester | | 0,015 g |
| Anisöl | | 0,05 g |
| Menthol | | 0,06 g |
| Ethanol rein | | 10,0 g |
| Wirkstoff | | 0,5 g |
| Natriumcyclamat | | 1,0 g |
| Glycerin | | 15,0 g |
| Dest. Wasser | ad | 100,0 ml |

Herstellungsverfahren

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

## Patentansprüche

1. Kondensierte Diazepinone der allgemeinen Formel I

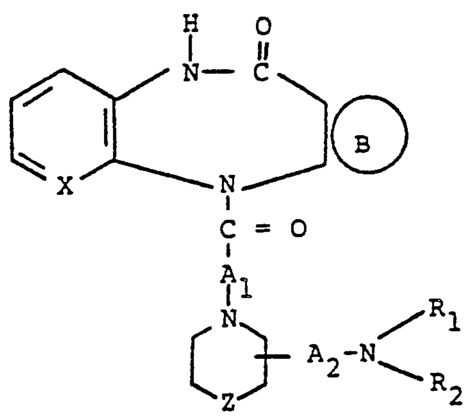

in der

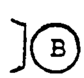

einen der zweiwertigen Reste

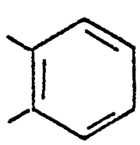

,

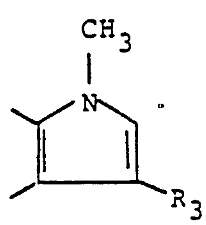

oder

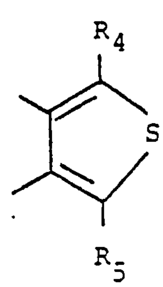

bedeutet und

X, $A_1$, $A_2$, $R_1$ bis $R_5$ und Z die folgenden Bedeutungen besitzen :

X ist die =CH-Gruppe oder, sofern

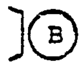

den ortho-Phenylenrest darstellt, auch ein Stickstoffatom ;

$A_1$ ist ein Alkylenrest mit 1 bis 2 Kohlenstoffatomen,

$A_2$ entweder ein Alkylenrest mit 1 bis 2 Kohlenstoffatomen, sofern er sich in der 2-Stellung zum Stickstoff des gesättigten heterocyclischen Ringes befindet oder, wenn er sich in der 3- oder 4-Stellung befindet, eine Einfachbindung oder die Methylengruppe,

$R_1$ ist ein verzweigter oder unverzweigter Alkylrest mit 1 bis 3 Kohlenstoffatomen,

$R_2$ ein verzweigter oder unverzweigter Alkylrest mit 1 bis 7 Kohlenstoffatomen, der gegebenenfalls noch an seinem 2. bis 7. Kohlenstoffatom durch eine Hydroxygruppe substituiert sein kann, ein Cycloalkyl- oder ein Cycloalkylmethylrest mit 3 bis 7 Kohlenstoffatomen im Ring, wobei der Cycloalkylring gegebenenfalls noch durch eine Hydroxygruppe substituiert sein kann,

$R_1$ und $R_2$ können aber auch zusammen mit dem dazwischenliegenden Stickstoffatom einen 4- bis 7-gliedrigen gesättigten, monocyclischen, heterocyclischen Ring bilden, der gegebenenfalls durch ein Sauerstoffatom oder durch die $N—CH_3$-Gruppe unterbrochen sein kann,

$R_3$ ist ein Wasserstoff- oder Chloratom oder die Methylgruppe,

$R_4$ ist ein Wasserstoffatom oder ein Alkylrest mit 1 bis 4 Kohlenstoffatomen,

$R_5$ ist ein Wasserstoff- oder Chloratom oder ein Alkylrest mit 1 bis 4 Kohlenstoffatomen und

Z entweder eine Einfachbindung oder ein Sauerstoffatom oder die Methylen- oder 1,2-Ethylengruppe,

und ihre $NR_1R_2$—N-Oxide, deren Diastereomere und Enantiomere und deren physiologisch verträgliche Säureadditionssalze mit anorganischen oder organischen Säuren.

2. Kondensierte Diazepinone der allgemeinen Formel I gemäß Anspruch 1, in der

$R_1$ bis $R_3$ und X wie im Anspruch 1 definiert sind,

Z eine Einfachbindung oder eine Methylengruppe,

$A_1$ und $A_2$ jeweils eine Methylengruppe,

$R_4$ ein Wasserstofftom oder eine Methylgruppe und $R_5$ ein Wasserstoff- oder. Chloratom oder eine Methylgruppe bedeuten,

und ihre $NR_1R_2$—N-Oxide,

deren Diastereomere und Enantiomere und deren physiologisch verträgliche Säureadditionssalze mit anorganischen oder organischen Säuren.

3. Kondensierte Diazepinone der allgemeinen Formel I gemäß Anspruch 1, in der

X wie im Anspruch 1 definiert ist,

Z eine Einfachbindung oder eine Methylengruppe,

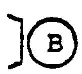

eine ortho-Phenylengruppe oder eine 3,4-verknüpfte Thienogruppe,

$A_1$ eine Methylengruppe,

$A_2$ eine Methylengruppe in 2-Stellung zum Stickstoffatom des gesättigten heterocyclischen Rings,

$R_1$ eine Methyl- oder Ethylgruppe und

$R_2$ eine Methyl- Ethyl- oder 4-Hydroxycyclohexylgruppe bedeuten,

und ihre $NR_1R_2$—N-Oxide,

deren Diastereomere und Enantiomere und deren physiologisch verträgliche Säureadditionssalze mit anorganischen oder organischen Säuren.

4. Arzneimittel, enthaltend eine Verbindung gemäß den Ansprüchen 1 bis 3 neben einem oder mehreren inerten Trägerstoffen und/oder Verdünnungsmitteln.

Verbindungen gemäß den Ansprüchen 1 bis 3. Kondensierte Diazepinone der allgemeinen Formel I gemäß Anspruch 1, in der

X wie im Anspruch 1 definiert ist,

Z eine Einfachbindung oder eine Methylengruppe,

eine ortho-Phenylengruppe oder eine 3,4-verknüpfte Thienogruppe,

$A_1$ eine Methylengruppe,

5. Verwendung von Verbindungen gemäß den Ansprüchen 1 bis 3 zur Herstellung von Arzneimitteln zur Behandlung von Bradycardien und Bradyarrhythmien.

6. Verfahren zur Herstellung der kondensierten Diazepinone gemäß den Ansprüchen 1 bis 3, dadurch gekennzeichnet, daß

a) zur Herstellung von basisch substituierten kondensierten Diazepinonen der allgemeinen Formel Ib,

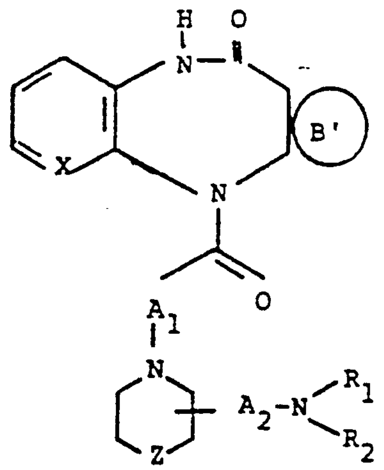

(Ib)

in der $R_1$, $R_2$, X, Z, $A_1$ und $A_2$ die in den Ansprüchen 1 bis 7 angegebenen Bedeutungen haben und

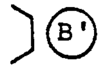

einen der zweiwertigen Reste

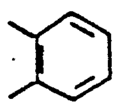

,

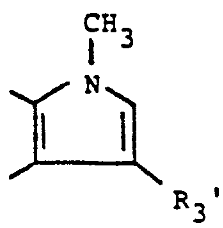

oder

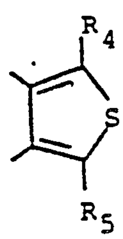

darstellt, wobei $R_4$ und $R_5$ wiederum die in den Ansprüchen 1 bis 7 gegebenen Bedeutungen annehmen können und $R_3'$ die Methylgruppe oder das Chloratom ist,

Halogenacylverbindungen der allgemeinen Formel II

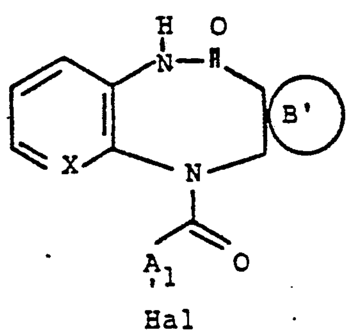

(II)

in der X,

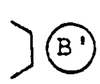

und $A_1$ die angegebenen Bedeutungen haben und Hal ein Chlor-, Brom- oder Jodatom ist, mit sekundären Aminen der allgemeinen Formel III,

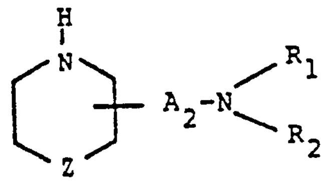

(III)

in der $R_1$, $R_2$, $A_2$ und Z die eingangs definierten Bedeutungen besitzen, umgesetzt werden oder

b) zur Herstellung von Verbindungen der vorstehenden allgemeinen Formel Ib, Tricyclen der allgemeinen Formel IV,

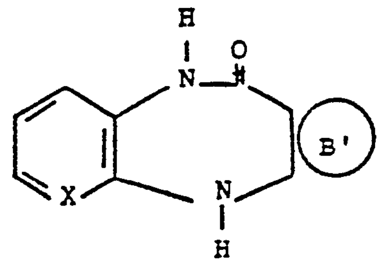

(IV)

worin

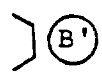

und X die oben angegebenen Bedeutungen haben, mit Carbonsäurederivaten der allgemeinen Formel V,

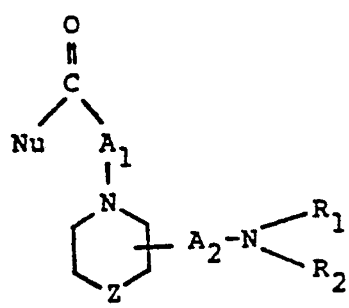

(V)

worin $R_1$, $R_2$, $A_1$, $A_2$ und Z die in den Ansprüchen 1 bis 7 angegebenen Bedeutungen haben und Nu eine nucleofuge Gruppe bzw. Abgangsgruppe darstellt, acyliert werden oder

c) zur Herstellung von Pyrrolobenzodiazepinonen der allgemeinen Formel Ic,

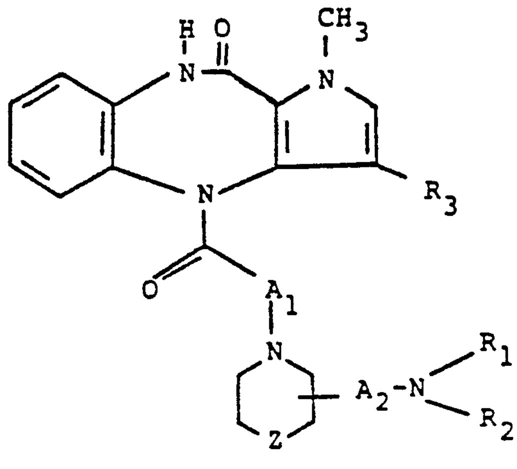

(Ic)

worin $R_1$, $R_2$ $A_1$, $A_2$ und Z die in den Ansprüchen 1 bis 7 erwähnten Bedeutungen haben und $R_3$ ein Wasserstoffatom ist, Verbindungen der allgemeinen Formel Ic, worin $R_3$ ein Chloratom bedeutet, hydrogenolysiert werden
und gewünschtenfalls eine so erhaltene Verbindung der allgemeinen Formel I zu ihrem $NR_1R_2$—N-Oxid oxidiert und/oder in ihre Diastereomeren und/oder Enantiomeren aufgetrennt und/oder in ihre physiologisch verträglichen Säureadditionssalze mit anorganischen oder organischen Säuren übergeführt wird.

7. Zwischenprodukte der allgemeinen Formel Ia,

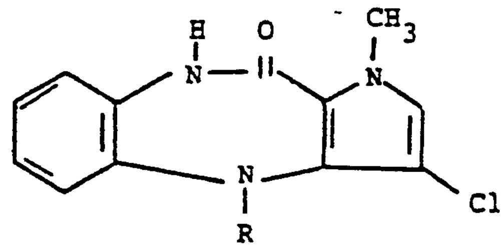

(Ia)

in der R das Wasserstoffatom oder einen Halogenacylrest, vorzugsweise einen Chloracylrest, mit ingesamt 2 oder 3 Kohlenstoffatomen bedeutet.

8. Verfahren zur Herstellung der der Formel Ia Zwischenprodukte gemäß Anspruch 7, dadurch gekennzeichnet, daß man Verbindungen der allgemeinen Formel VI

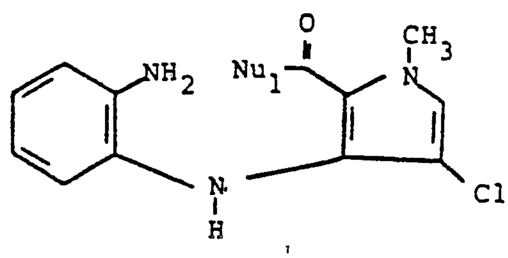

(VI)

in der $Nu_1$ eine geeignete nucleofuge Gruppe darstellt, cyclisiert und gegebenenfalls anschließend den erhaltenen Tricyclus der allgemeinen Formel Ia, in der R das Wasserstoffatom bedeutet, halogenacyliert.

9. Verfahren gemäß Anspruch 6a, dadurch gekennzeichnet, daß die Halogenacylverbindungen der allgemeinen Formel II mit den sekundären Aminen der allgemeinen Formel III in einem inerten Lösungsmittel bei Temperaturen zwischen —10 °C und der Siedetemperatur des Lösungsmittels, gegebenenfalls in Gegenwart einer Hilfsbase oder eines Überschusses des Amins der allgemeinen Formel III, umgesetzt werden, gegebenenfalls aber zuerst aus der Halogenacylverbindung der allgemeinen Formel II durch die Einwirkung der Base ein Molekül H-Hal abgespalten und anschließend die so gebildete Acryloylverbindung mit dem Amin der allgemeinen Formel III umgesetzt wird.

10. Verfahren gemäß Anspruch 6b, dadurch gekennzeichnet, daß die Tricyclen der allgemeinen Formel IV mit den Carbonsäurederivaten der allgemeinen Formel V, vorzugsweise mit den Säurehalogeniden, -estern, -anhydriden oder gemischten -anhydriden oder N-Alkyl-2-acyloxypyridiniumsalzen dieser den Carbonsäurederivaten zugrundeliegenden Carbonsäuren, bei Temperaturen zwischen —25 °C und 130 °C in einem inerten Lösungsmittel umgesetzt werden, gegebenenfalls in Gegenwart eines säurebindenden Mittels.

11. Verfahren gemäß Anspruch 6c, dadurch gekennzeichnet, daß die Hydrogenolyse einer Verbindung der allgemeinen Formel Ic in Gegenwart von Katalysatoren der Metalle der VIII. Nebengruppe des Periodensystems bei Wasserstoffdrucken von 1 bis 300 bar und Temperaturen von 0 bis 130 °C in Anwesenheit von Lösungsmitteln durchgeführt wird.

## Claims

1. Condensed diazepinones of general formula I

(See formula I p.39)

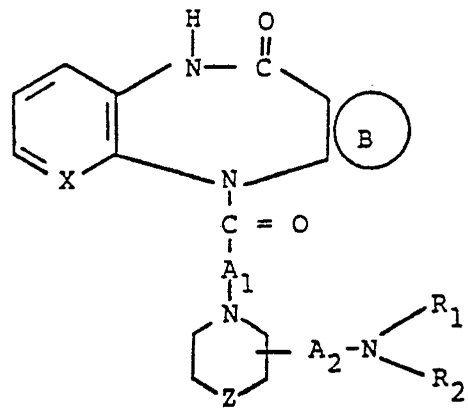

(I)

wherein

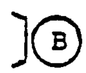

represents one of the divalent groups

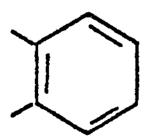 , 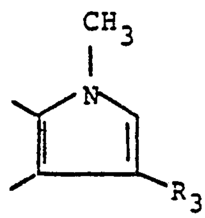 oder 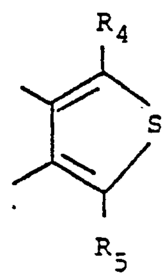

and

X, $A_1$, $A_2$, $R_1$ to $R_5$ and Z are defined as follows :

X is the =CH group or, if

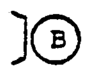

represents the ortho-phenylene group, it may also represent a nitrogen atom ;

$A_1$ is an alkylene group with 1 to 2 carbon atoms,

$A_2$ either represents an alkylene group with 1 to 2 carbon atoms, if it is in the 2-position relative to the nitrogen of the saturated heterocyclic ring or, if it is in the 3- or 4-position, it represents a single bond or the methylene group,

$R_1$ is a branched or unbranched alkyl group with 1 to 3 carbon atoms,

$R_2$ represents a branched or unbranched alkyl group with 1 to 7 carbon atoms which may optionally also be substituted at its 2nd to 7th carbon atom by a hydroxy group, a cycloalkyl or cycloalkylmethyl group with 3 to 7 carbon atoms in the ring, the cycloalkyl ring optionally further being substituted by a hydroxy group,

$R_1$ and $R_2$ may, however, also form together with the nitrogen atom between them a 4- to 7-membered saturated, monocyclic, heterocyclic ring which may optionally be interrupted by an oxygen atom or by the $N—CH_3$ group,

$R_3$ is a hydrogen or chlorine atom or a methyl group,

$R_4$ is a hydrogen atom or an alkyl group with 1 to 4 carbon atoms,

$R_5$ is a hydrogen or chlorine atom or an alkyl group with 1 to 4 carbon atoms and

Z represents either a single bond or an oxygen atom or the methylene or 1,2-ethylene group,

and the $NR_1R_2—N$ oxides thereof, the diastereoisomers and enantiomers and physiologically acceptable acid addition salts thereof with inorganic or organic acids.

2. Condensed diazepinones of general formula I as claimed in claim 1, wherein

$R_1$ to $R_3$ and X are defined as in claim 1,

Z represents a single bond or a methylene group,

$A_1$ and $A_2$ each represent a methylene group,

$R_4$ represents a hydrogen atom or a methyl group and

$R_5$ represents a hydrogen or chlorine atom or a methyl group,

and the $NR_1R_2—N$ oxides thereof,

the diastereoisomers and enantiomers and physiologically acceptable acid addition salts thereof with inorganic or organic acids.

3. Condensed diazepinones of general formula I as claimed in claim 1, wherein
X is defined as in claim 1,
Z represents a single bond or a methylene group,

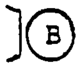

represents an ortho-phenylene group or a 3,4-linked thieno group,
$A_1$ represents a methylene group,
$A_2$ represents a methylene group in the 2-position relative to the nitrogen atom of the saturated heterocyclic ring,
$R_1$ represents a methyl or ethyl group and
$R_2$ represents a methyl, ethyl or 4-hydroxycyclohexyl group,
and the $NR_1R_2$—N-oxides thereof,
the diastereoisomers and enantiomers and physiologically acceptable acid addition salts thereof with inorganic or organic acids.

4. Pharmaceutical compositions containing a compound as claimed in claims 1 to 3 together with one or more inert carriers and/or diluents.

5. Use of compounds as claimed in claims 1 to 3 for the preparation of pharmaceutical compositions for the treatment of bradycardia and bradyarrhythmia.

6. Process for preparing condensed diazepinones as claimed in claims 1 to 3, characterised in that
a) in order to prepare basically substituted condensed diazepinones of general formula Ib,

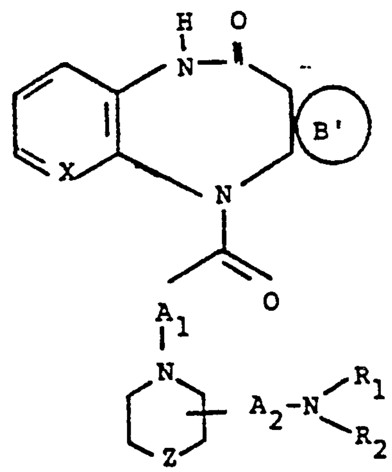

wherein $R_1$, $R_2$, X, Z, $A_1$ and $A_2$ are defined as in claims 1 to 7 and

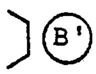

represents one of the divalent groups

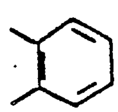

,

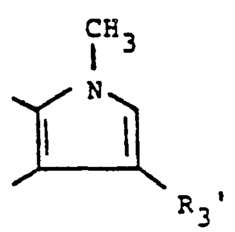

or

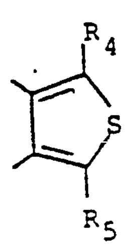

wherein $R_4$ and $R_5$ may again assume the meanings given in claims 1 to 7 and $R_3'$ is the methyl group or the chlorine atom, haloacyl compounds of general formula II

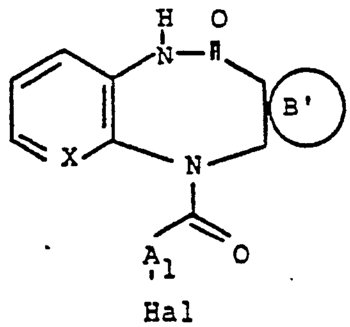

(II)

wherein X,

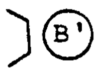

and $A_1$ are defined as hereinbefore and Hal is a chlorine, bromine or iodine atom, are reacted with secondary amines of general formula III,

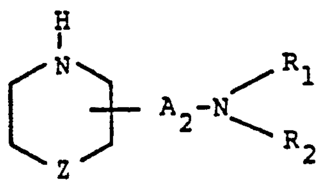

(III)

wherein $R_1$, $R_2$, $A_2$ and Z are defined as hereinbefore, or

b) in order to prepare compounds of general formula Ib above, tricyclic compounds of general formula IV,

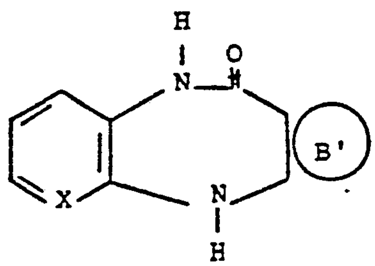

(IV)

wherein

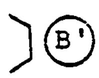

and X are defined as hereinbefore, are acylated with carboxylic acid derivatives of general formula V

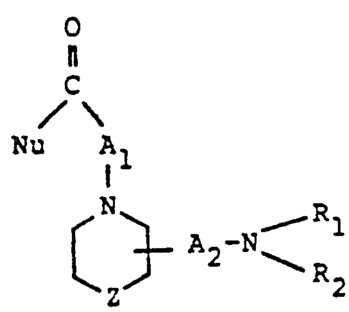

(V)

wherein $R_1$, $R_2$, $A_1$, $A_2$ and Z have the meanings given in claims 1 to 7 and Nu represents a nucleophobic group or leaving group, or

c) in order to prepare pyrrolobenzodiazepinones of general formula Ic

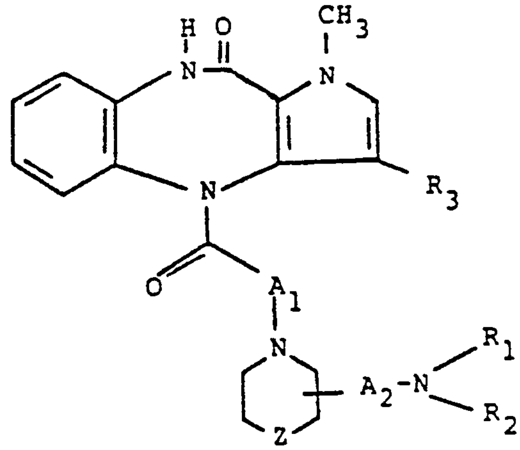

(Ic)

wherein $R_1$, $R_2$, $A_1$, $A_2$ and Z have the meanings given in claims 1 to 7 and $R_3$ is a hydrogen atom, compounds of general formula Ic wherein $R_3$ represents a chlorine atom are hydrogenolysed and if desired a compound of general formula I thus obtained is oxidised into the $NR_1R_2$—N oxide thereof and/or resolved into its diastereoisomers and/or enantiomers and/or converted into the physiologically acceptable acid addition salts thereof with inorganic or organic acids.

7. Intermediate products of general formula Ia

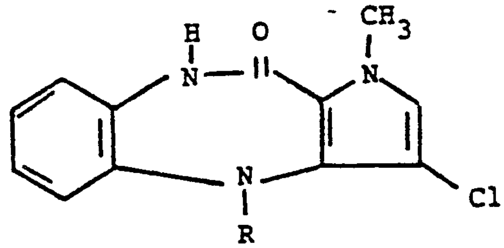

(Ia)

wherein R represents a hydrogen atom or a haloacyl group, preferably a chloroacyl group, having a total of 2 or 3 carbon atoms.

8. Process for preparing the intermediate products of formula Ia as claimed in claim 7, characterised in that compounds of general formula VI

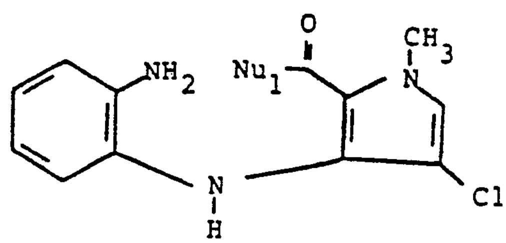

(VI)

wherein $Nu_1$ represents a suitable nucleophobic group, are cyclised and subsequently, if desired, the resulting tricyclic compound of general formula Ia wherein R represents a hydrogen atom is haloacylated.

9. Process as claimed in claim 6a, characterised in that the haloacyl compounds of general formula II are reacted with the secondary amines of general formula III in an inert solvent at temperatures between —10°C and the boiling temperature of the solvent, optionally in the presence of an auxiliary base or an excess of the amine of general formula III, but first of all, if desired, a H-Hal molecule is split off from the haloacyl compound of general formula II by the action of the base and subsequently the acryloyl compound thus formed is reacted with the amine of general formula III.

10. Process as claimed in claim 6b, characterised in that the tricyclic compounds of formula IV are reacted with the carboxylic acid derivatives of general formula V, preferably with the acid halides, esters, anhydrides or mixed anhydrides or N-alkyl-2-acyloxypyridinium salts of these carboxylic acids on which the carboxylic acid derivatives are based, at temperatures of between —25 °C and 130 °C in an inert solvent, optionally in the presence of an acid binding agent.

11. Process as claimed in claim 6c, characterised in that the hydrogenolysis of a compound of general formula Ic is carried out in the presence of catalysts of metals of the VIIIth sub-group of the Periodic Table at hydrogen pressures of from 1 to 300 bar and at temperatures of from 0 to 130 °C in the presence of solvents.

**Revendications**

1. Diazépinones condensées de formule générale I

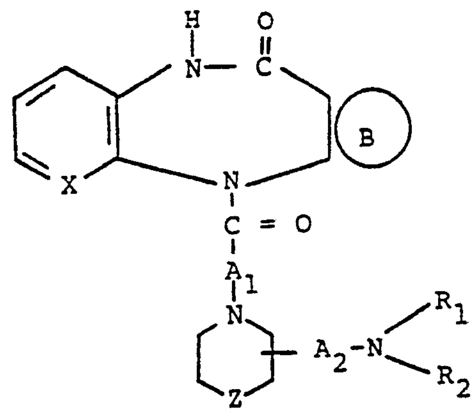

(I)

dans laquelle

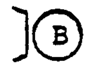

représente l'un des radicaux bivalents

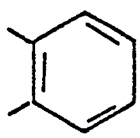

,

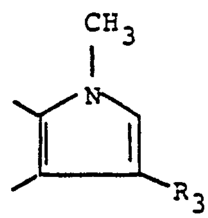

ou

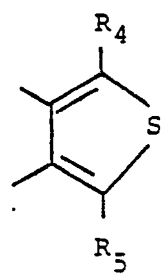

et

X, $A_1$, $A_2$, $R_1$ à $R_5$ et Z possèdent les significations suivantes :

X est le groupe = CH— ou, dans la mesure où

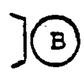

représente le radical ortho-phénylène, également un atome d'azote ;

$A_1$ est un radical alcoylène avec 1 à 2 atomes de carbone ;

$A_2$ représente soit un radical alcoylène avec 1 à 2 atomes de carbone, dans la mesure où il se trouve en position 2 par rapport à l'azote du cycle hétérocyclique saturé soit, lorsqu'il se trouve en position 3 ou 4, une liaison simple ou le groupe méthylène ;

$R_1$ est un radical alcoyle avec 1 à 3 atomes de carbone, ramifié ou non ramifié ;

$R_2$ représente un radical alcoyle avec 1 à 7 atomes de carbone, ramifié ou non ramifié, qui peut éventuellement être encore substitué sur son deuxième à septième atome de carbone par un groupe hydroxy, ou par un radical cycloalcoyle ou un radical cycloalcoylméthyle avec 3 à 7 atomes de carbone dans le cycle, le cycle cycloalcoyle pouvant éventuellement être encore substitué par un groupe hydroxy ;

$R_1$ et $R_2$ peuvent cependant également former ensemble avec l'atome d'azote intermédiaire un cycle hétérocyclique, monocyclique, saturé à 4 à 7 membres qui peut éventuellement être interrompu par un atome d'oxygène ou par le groupe N—$CH_3$— ;

$R_3$ est un atome d'hydrogène ou de chlore ou le groupe méthyle ;

$R_4$ est un atome d'hydrogène ou un radical alcoyle avec 1 à 4 atomes de carbone ;

$R_5$ est un atome d'hydrogène ou de chlore ou un radical alcoyle avec 1 à 4 atomes de carbone ; et

Z représente soit une liaison simple, soit un atome d'oxygène soit le groupe méthylène ou 1,2-éthylène ; et leurs $NR_1R_2$—N-oxydes,
leurs diastéréo-isomères et énantiomères et leurs sels d'addition d'acides physiologiquement supportables avec des acides minéraux ou organiques.

2. Diazépinones condensées de formule générale I selon la revendication 1, dans laquelle
$R_1$ à $R_3$ et X sont définis comme dans la revendication 1,
Z représente une liaison simple ou un groupe méthylène,
$A_1$ et $A_2$ représentent chacun un groupe méthylène,
$R_4$ représente un atome d'hydrogène ou un groupe méthyle et
$R_5$ représente un atome d'hydrogène ou de chlore ou un groupe méthyle,
et leurs $NR_1R_2$—N-oxydes,
leurs diastéréo-isomères et énantiomères et leurs sels d'addition d'acides physiologiquement supportables avec des acides minéraux ou organiques.

3. Diazépinones condensées de formule générale I selon la revendication 1, dans laquelle
X est défini comme à la revendication 1,
Z représente une liaison simple ou un groupe méthylène,

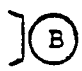

représente un groupe ortho-phénylène ou un groupe thiéno lié en position 3, 4,
$A_1$ représente un groupe méthylène,
$A_2$ représente un groupe méthylène en position 2 par rapport à l'atome d'azote du cycle hétérocyclique saturé,
$R_1$ représente un groupe méthyle ou éthyle, et
$R_2$ représente un groupe méthyle, éthyle ou 4-hydroxycyclohexyle,
et leurs $NR_1R_2$—N-oxydes
leurs diastéréo-isomères et énantiomères et leurs sels d'addition d'acides physiologiquement supportables avec des acides minéraux ou organiques.

4. Médicament contenant un composé selon les revendications 1 à 3 conjointement à un ou plusieurs excipients et/ou diluants inertes.

5. Utilisation des composés selon les revendications 1 à 3 pour la fabrication de médicaments pour le traitement des bradycardies et des bradyarythmies.

6. Procédé pour la préparation des diazépinones condensées selon les revendications 1 à 3, caractérisé en ce que

a) pour la préparation de diazépinones condensées basiquement substituées, de formule générale Ib

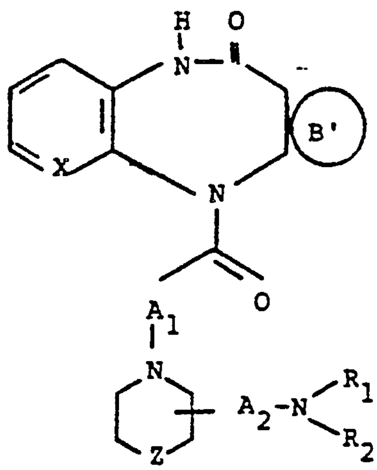

dans laquelle $R_1$, $R_2$, X, Z, $A_1$ et $A_2$ ont les significations indiquées dans les revendications 1 à 7, et

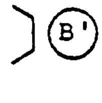

représente l'un des radicaux bivalents

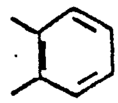 , 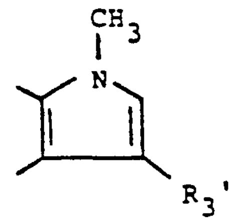

ou

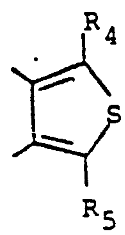

$R_4$ et $R_5$ pouvant de nouveau prendre les significations données dans les revendications 1 à 7 et $R_3'$ étant le groupe méthyle ou l'atome de chlore,

On fait réagir des composés halogénoacylés de formule générale II

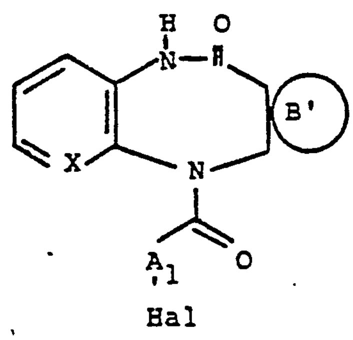

(II)

dans laquelle X,

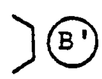

et $A_1$ ont les significations indiquées et Hal est un atome de chlore, de brome ou d'iode, avec des amines secondaires de formule générale III

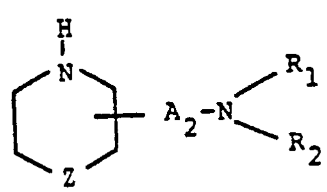

(III)

dans laquelle $R_1$, $R_2$, $A_2$ et Z possèdent les significations définies au début ou

b) pour la préparation de composés de formule générale Ib ci-dessus, on acyle des tricycles de formule générale IV

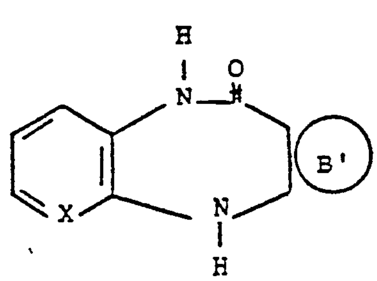

(IV)

dans laquelle

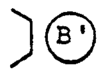

et X ont les significations indiquées plus haut, au moyen de dérivés d'acides carboxyliques de formule générale V

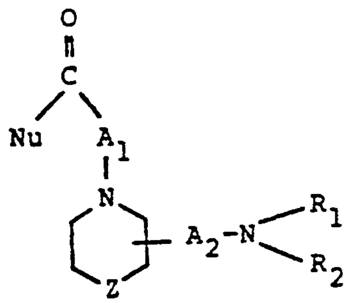

(V)

dans laquelle $R_1$, $R_2$, $A_1$, $A_2$ et Z ont les significations indiquées dans les revendications 1 à 7, et Nu représente un groupe nucléofuge ou groupe partant, ou

c) pour la préparation de pyrrolobenzodiazépinones de formule générale Ic

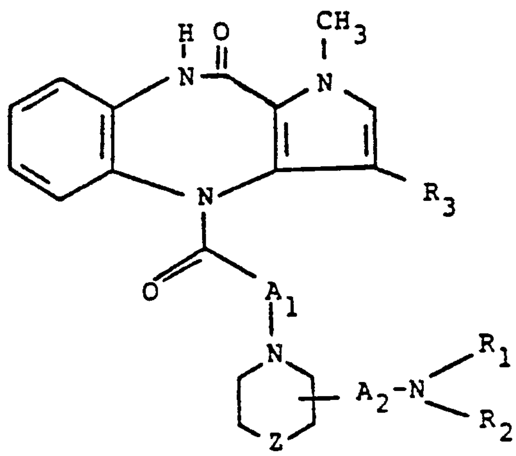

(Ic)

dans laquelle $R_1$, $R_2$, $A_1$, $A_2$ et Z ont les significations mentionnées dans les revendications 1 à 7 et $R_3$ est un atome d'hydrogène, on hydrogénolyse des composés de formule générale Ic dans laquelle $R_3$ représente un atome de chlore,

et si on le désire, on oxyde un composé de formule générale I ainsi obtenu en son $NR_1R_2$—N-oxyde et/ou on le sépare en ses diastéréo-isomères et/ou énantiomères et/ou on le transforme en ses sels d'addition d'acides physiologiquement supportables avec des acides minéraux ou organiques.

7. Produits intermédiaires de formule générale Ia

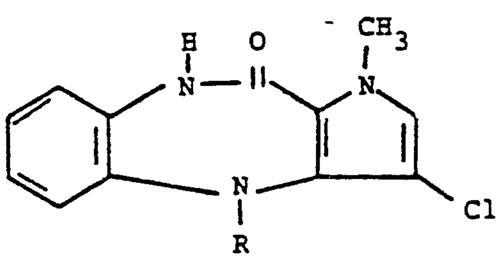

(Ia)

dans laquelle R représente l'atome d'hydrogène ou un radical halogéno-acyle, de préférence un radical chloroacyle, avec en tout 2 ou 3 atomes de carbone.

8. Procédé pour la préparation des produits intermédiaires de formule Ia selon la revendication 7, caractérisé en ce qu'on cyclise des composés de formule générale VI

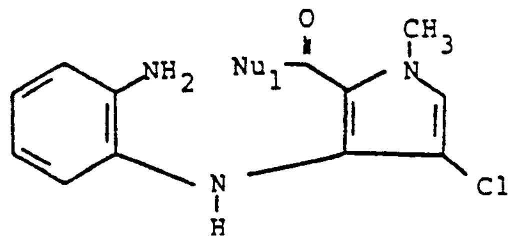

(VI)

dans laquelle $Nu_1$ représente un groupe nucléofuge approprié et éventuellement on halogénoacyle ensuite le tricycle obtenu de formule générale Ia, dans laquelle R représente l'atome d'hydrogène.

9. Procédé selon la revendication 6a, caractérisé en ce qu'on fait réagir les composés halogénoacylés de formule générale II avec les amines secondaires de formule générale III dans un solvant inerte à des températures entre — 10 °C et la température d'ébullition du solvant, éventuellement en présence d'une base adjuvante ou d'un excès de l'amine de formule générale III, éventuellement toutefois d'abord on clive, à partir du composé halogénoacylé de formule générale II une molécule H-Hal par action de la base et on fait réagir ensuite le composé acryloylé ainsi formé avec l'amine de formule générale III.

10. Procédé selon la revendication 6b, caractérisé en ce qu'on fait réagir les tricycles de formule générale IV avec les dérivés d'acides carboxyliques de formule générale V, de préférence avec les halogénures, esters, anhydrides ou anhydrides mixtes d'acides ou avec des sels de N-alcoyl-2-acyloxypyridinium, de ces acides carboxyliques à la base des dérivés d'acides carboxyliques, à des températures entre — 25 °C et 130 °C dans un solvant inerte, éventuellement en présence d'un agent fixant les acides.

11. Procédé selon la revendication 6c, caractérisé en ce que l'hydrogénolyse d'un composé de formule générale Ic est effectuée en présence de catalyseurs à base de métaux du sous-groupe VIII du système de classification périodique des éléments sous des pressions d'hydrogène de 1 à 300 bars et à des températures de 0 à 130 °C en présence de solvants.